# EUROPEAN PATENT APPLICATION

(11) **EP 0 712 608 A2**
(43) Date of publication of application: **22.05.1996**
(21) Application number: 95117618.9
(22) Date of filing: 08.11.1995
(51) Int. Cl.: A61B 17/28, A61B 10/00, A61B 17/32

(54) **Surgical instrument for operation**

(30) Priority: 11.11.1994 JP 278002/94
(71) Applicant: OLYMPUS OPTICAL CO., LTD., Tokyo 151 (JP)
(72) Inventor: Sugai, Toshiya, c/o Olympus Optical Co., Ltd, Hachioji-shi, Tokyo (JP); Bito, Shiro, c/o Olympus Optical Co., Ltd, Hachioji-shi, Tokyo (JP); Iida, Koji, c/o Olympus Optical Co., Ltd, Hachioji-shi, Tokyo (JP); Kikuchi, Yasuhiko, c/o Olympus Optical Co., Ltd, Hachioji-shi, Tokyo (JP); Kaji, Kunihide, c/o Olympus Optical Co., Ltd, Hachioji-shi, Tokyo (JP)
(74) Representative: Grünecker, Kinkeldey, Stockmair & Schwanhäusser Anwaltssozietät

(57) **Abstract**

A surgical instrument for operation purposes has an operation section which an operator operates, a treatment section which is inserted into a living body and treats tissue, an insertion section which has a first end disposed on the operation section side and a second end disposed on the treatment section side, and a treatment section drive mechanism which is operatively engaged between the operation section and the treatment section and drives the treatment section in response to the operation of the operation section. At least one of members which form the treatment section and the treatment section drive mechanism is manufactured of metallic material by forging.

## Description

### BACKGROUND OF THE INVENTION

### Field of the Invention:

The present invention relates to a surgical instrument for operation purposes which is used in inspection or operation using an endscope and treats tissue.

### Descussion of the Related Arts:

There is a publicly known surgical instrument for operation purposes which is inserted into a body cavity like an endoscope to treat tissue when inspection or operation is carried out using an endoscope. The surgical instrument for use in operation has an insertion portion to be inserted into a living body, a treatment portion provided at the leading end of the insertion portion which treats tissue of the living body, and an actuation portion provided at an area closer to a handle of the instrument for actuating the treatment member. Conventionally, a treatment member of the surgical instrument of this type, or components of a treatment member drive mechanism for driving the treatment member are usually manufactured by machining.

For example, Unexamined Japanese Patent Application Nos. Hei. 6-179049 and Hei. 5-48245, filed by the present applicants, disclose forceps having a complicated structure of a surgical treatment instrument in which a plurality of teeth are formed in the treatment member. Further, U.S. Patent 5,234,453 discloses forceps in which cobalt-series alloy is cast into teeth of forceps.

According to the surgical instrument for operation purposes having the above mentioned construction, components of the treatment member and drive mechanism for driving the treatment member are generally manufactured by machining. For this reason, in the case of forceps having the complicated structure like a surgical treatment instrument in which a plurality of teeth are formed in the treatment member as disclosed in Japanese Patent Application Nos. Hei. 6-179049 and Hei. 5-48245, it often takes much expense in time and effort to mold the teeth of the forceps. Particularly, this makes it difficult to reduce cost when mass-producing forceps.

A connecting area between the treatment member and coupling means of the treatment member drive mechanism is apt to become thin and, hence, there is a possibility that the connecting area between the treatment member and the coupling means of the treatment member drive mechanism fractures when an excessive force exerts, as an operating physical force, on the connecting area. Particularly in recent years, it is desired to reduce the diameter of the insertion portion to a much greater extent in order to alleviate a heavy physical burden of the patient, which in turn makes the problem of strength of the forceps more pronounced.

If cobalt-series alloy is cast into teeth of the forceps as disclosed in U.S. Patent 5,234,453, it is possible to inexpensively mass-produce a treatment member having a complicated structure while retaining the mechanical strength of the treatment member. However, the thus cast treatment member has a rough surface, just as it is. If the treatment member of this type is used as a disposable treatment member which is usable just for once. Poorer quality of the cast treatment member as merchandize, the rough surface of the treatment member, poor actuation of movable portions of the treatment member, and poor cleansing characteristics present only trivial problems because the treatment member is disposable. In contrast, if the treatment member of this type is used as a reusable article which is used several times by repeatedly cleansing and sterilizing the treatment member, the cast treatment member is usually subjected to surface treatment by, for example, a burnisher to satisfy user demands for a high-quality treatment member of this type and the necessity for superior cleansing characteristics. In effect, the casting of cobalt-based alloy to a treatment member does not lead to much cost cutting.

An operating physical force for actuating means which results from the use of an operating member such as a handle is generally transmitted to the treatment member drive mechanism through a rod-shaped transmission member. In many cases, a transducer in which a coupling member molded into a ball shape is slidably connected to the inside of the operating member is employed for a transducing mechanism made up of the operating member of the actuating means and the rod-shaped transmission member. However, in this case, undesirable bite lock likely occurs between the ball-shaped coupling member and the operating member, which in turn might break the root of the ball-shaped coupling member. In this way, the treatment member made of cobalt-series alloy has a problem in durability.

As with conventional surgical instruments, if the constituent members of the treatment member drive mechanism are manufactured by machining or casting, the strength of the constituent members will always be lower than the strength of materials used for the constituent members. For this reason, it is inevitably necessary to design a treatment member drive mechanism with special attention to the strength thereof by increasing the thickness of each of constituent members of the treatment member drive mechanism to increase the mechanical strength of the constituent members. This improvement on the mechanical strength of the constituent members hinders size and weight reductions of each of the constituent members of the treatment member drive mechanism.

The outer diameter of an insertion portion of a surgical instrument for operation is usually extremely small and is set to, for example, 5mm⌀ to 10mm⌀ or thereabouts. It is difficult to arrange constituent members of the treatment member drive mechanism within such a limited space of the insertion portion. A degree of freedom of design is considerably restricted in order to retain the mechanical strength of each of the constituent members of the treatment member drive mechanism. Most of the internal space of the surgical instrument is occupied by the constituent members of the treatment member drive mechanism. For this reason, it is difficult to effectively cleanse gaps between the constituent members of the treatment member drive mechanism, and also it takes a lot of time to cleanse and sterilize the constituent members.

### SUMMARY OF THE INVENTION

The present invention was made in view of the foregoing drawbacks accompanying the conventional art, and an object of the present invention is to provide a surgical instrument for operation purposes which provides improved productivity of a treatment member and constituent members of a treatment member drive mechanism, cost reduction, enhanced elasticity, ensured design freedom, and ease of cleansing and sterilization of the inside of an insertion portion without reducing the mechanical strength thereof.

The above and other objects can be achieved by a provision of a surgical instrument for operation which, according to the present invention, includes an insertion section to be inserted into a living body, a treatment member disposed at the leading end of the insertion section for treating tissue, operating means connected to an area of the insertion section closer to a handle of the instrument for actuating the treatment member, and a treatment member drive mechanism which drives the treatment member by the actuation of the operating means, wherein at least one of constituent members of the treatment member or the treatment member drive mechanism is made by forging metallic material.

As a result of producing at least one of the constituent members of the treatment member or the treatment member drive mechanism from forged parts made of metallic material, machining required to manufacture the surgical instrument is reduced, thereby resulting in reduced labor for manufacturing, improved productivity, and reductions in manufacturing cost. As disclosed in various engineering books, for example, "Theory of Plasticity, and Plastic Working for University Course by Masujiro Hayama" and "Course of Asakura Mechanical Engineering 12: Machine Manufacturing Method by Yoshiro Abu and Hiroshi Tamura", the mechanical strength, elasticity, and toughness of members are increased by improving the characteristics of material itself utilizing forging, thereby resulting in enhanced degree of freedom of design and durability. Associated with an increase in mechanical strength of the constituent members as a result of forging, the size of the constituent members is reduced, which in turn ensures a relatively wide internal space in the insertion section of the surgical instrument. As a result of this, the cleansing and sterilization characteristics of the surgical instrument are improved.

According to a first preferred aspect of the present invention, a surgical instrument for operation purposes comprises (a) operation means, (b) an insertion section, with one end thereof being connected to the operation means, having at the other end thereof a treatment section for treating tissue, and the insertion section inserting the treatment section into a living body and an endoscope, (c) at least one or more treatment member for moving the treatment section, between a first position where the treatment section does not obstruct the insertion and removal of the treatment section and a second position where the treatment section treats the tissue, at least when the surgical instrument is inserted into, and removed from, the living body or the endoscope, (d) a treatment member drive mechanism for driving the treatment member in response to the operation of the operation means, and (e) the treatment member drive mechanism and at least one or more treatment member, or the treatment member drive mechanism or at least one or more treat member being manufactured by forging metallic materials.

According to a second preferred aspect of the present invention, in the surgical instrument for operation purposes as defined in the first preferred aspect, the operation means comprises (a) two movable operation members being movable in a longitudinal direction of the insertion section, and (b) means for moving the treatment member drive mechanism in response to the operation of the movable operation members and driving the treatment member as a result of the movement of the treatment drive mechanism.

According to a third preferred aspect of the present invention, in the surgical instrument for operation purposes as defined in the first preferred aspect, the operation section comprises (a) a fixed operation member being fixed with respect to a longitudinal direction of the insertion section, and a movable operation member being movable with respect to the fixed operation member for operating the treatment member.

According to a fourth preferred aspect of the present invention, in the surgical instrument for operation purposes as defined in any one of the first to third preferred aspects, the treatment member drive mechanism comprises (a) transmission means disposed within the insertion section for transmitting the motion of the operation means to the treatment member, (b) conversion means for converting the motion of the operation means into the movement of the transmission means, and (c) connection means for transmitting the movement of the transmission means to the treatment member.

According to a fifth preferred aspect of the present invention, in the surgical instrument for operation purposes as defined in any one of the first to fourth preferred aspects, the connection means is made of (a) at least one or more cam mechanism which is made up of cam grooves and a cum pin engaging with the cam grooves.

Like a device as disclosed in EPA-543,107 A2, if a cam mechanism is employed for the treatment member drive mechanism, the cam mechanism is apt to undergo abrasion due to friction and bite lock between the cam grooves and the cam pin. To solve these problems, as disclosed in Japanese Patent Application No. Hei. 5-104633, the surface of a member is covered with a TiN coating as a CVD coating, and the coating is buffed to make the surface hard and decrease a frictional coefficient. However, this finishing method effectively solve the above mentioned problem, but it costs much.

The fifth aspect of the present invention is directed to solve the above mentioned prior art problems such as the abrasion of the cam mechanism and the bite lock between the cam grooves and the cam pin occurred when the cam mechanism is used for the treatment member drive mechanism without the increase of cost.

By virtue of the configuration as defined in the fifth aspect of the present invention, in addition to the elimination of the above mentioned problems, the surface hardness of the member is improved by forging, and the abrasion of the cam mechanism and the bite lock between the cam grooves and the cam pin became less likely to occur. Further, the material itself of the member was made even and dense, and hence it is possible to finish the surface more smoothly, thereby resulting in a reduced frictional coefficient. These improvements are made on the member during the course of molding, and therefore surface processing of the member becomes unnecessary, which contributes to prevention of a rise in cost.

According to a sixth preferred aspect of the present invention, in the surgical instrument for operation purposes as defined in any one of the first to fourth preferred aspects, the connection means comprises at least one or more a set of link mechanisms.

Like treatment member drive mechanisms for which a link mechanism is used, as disclosed in Japanese Patent Application No. Hei. 5-48245, U.S. Patent 5,234,453, and PCT WO91/02493, members which form the link mechanism are small in size, and hence it is difficult to ensure the mechanical strength of the members. Further, these members are made by machining one by one during manufacturing steps, which results in poor productivity and increased cost.

The sixth aspect of the present invention is directed to solve these drawbacks in the prior art. Specifically, the sixth aspect of the invention is intended to solve the problems, that is, insufficient mechanical strength of members of a link mechanism caused when the link mechanism is used for the treatment member drive mechanism for reasons of minuteness of the members, and poor productivity and increased cost attributable to the manufacture of very small members by machining.

By virtue of the configuration as defined in the sixth aspect, members of the link mechanism of the treatment member drive mechanism are made by forging metallic materials, and hence members having a larger hardness are obtained compared with members manufactured by machining. Moreover, the machining of small members one by one becomes unnecessary, and, for example, it is also possible to produce several tens of members at one time using one mold, which results in improved productivity and reduced cost.

According to a seventh preferred aspect of the present invention, in the surgical instrument for operation purposes as defined in any one of the first to sixth preferred aspects, the treatment member has (a) a proximal end connected to a connection mechanism and a distal end opposite to the proximal end, and (b) an intermediate portion, between the proximal end and the distal end, which is made by forging so as to possess elasticity.

According to an eighth preferred aspect of the present invention, in the surgical instrument for operation purposes as defined in any one of the first to seventh preferred aspects, the treatment member comprises (a) a set of treatment members which are opposite to each other, (b) the treatment members being supported by the treatment section connected to the distal end of the insertion section in such a way as to pivotally move around a support shaft, (c) the treatment members which relatively pivot around the support shaft between at least one close position and at least one open position, and (d) the treatment members being previously curved in such a way that the distal ends of the treatment members first come into contact with each other when the treatment members are pushed to the close position.

Like surgical forceps as disclosed in USP3,101,715, in the case of general surgical forceps, forceps are made by forging, whereby an excessive grasping force occurred when tissue is grasped is absorbed by the deformation of teeth of the forceps or deformation of the teeth of the forceps in agreement with the shape of the tissue to be grasped. Thus, the grasping of the tissue without damage is commonly practiced. However, as previously mentioned, in the case of the surgical instrument for operation purposes, a treatment member is manufactured by machining or casting. In general, stainless steel is used when the treatment member is made by machining, whereas cobalt-series materials are used when the treatment member is made by casting. For these reasons, materials having large elasticity like springs are not available. For reasons of restrictions such as materials suitable for machining and the characteristics of members made by casting, a resultant member has less elasticity. When tissue was grasped by forceps using such a member, it was impossible for the forceps to absorb an excessive grasping force by means of the elasticity of the treatment member.

To improve the forceps with respect to this point, like surgical instruments for operation purposes, as disclosed in PCT WO 91/02493 and EPO 543 107 A2, an elastic plate material was used for a treatment member. However, in the case of the plate material, the plate material is machined to obtain elasticity, whereby the thickness of the plate material becomes small. Moreover, the plate material has edges as a result of the machining, and these edges may cause damage to the tissue. In this way, the plate material was not effective means to eliminate the drawbacks in the prior art. Further, in the case of a surgical instrument having such a treatment member, separation operation which is very important in an operation was impossible for reasons of the shape or characteristics of the treatment member. Furthermore, since the treatment member is formed from plate material, it was impossible to manufacture a surgical instrument like so-called intestine forceps such as disclosed in U.S. Patent 3,101,715.

In the case of a retractable surgical instrument as disclosed in U.S. Patent 5,222,973, the deflection of a treatment member realized the grasping of tissue without damage. However, in the case of general retractable type forceps, for reasons of user's taste or familiarity, not all of forceps are of retractable type.

The seventh and eighth aspects of the present invention are directed to solve the problems in the prior art, namely, the difficult of deformation of a treatment member which makes it impossible to absorb an excessive grasping force, and possible damage to tissue by edges of a plate material occurred if the plate material is used for the treatment member to make it possible for the treatment member to deform.

By virtue of the configurations as defined in the seventh and eighth aspects of the present invention, if the treatment member is made by forging in such a way that a fiber flow appears in a longitudinal direction of the treatment member, it is possible to provide the treatment member with effective elasticity without changing the conventional configuration of the treatment member. Hence, dislike a treatment member formed from a plate material which may cause damage to tissue, the treatment member manufactured according to this invention can realize the grasping of tissue without damage by absorbing an excessive grasping force. Further, the treatment member manufactured by the present invention is the same in shape as the conventional treatment member, and therefore a treatment member drive mechanism having the same configuration may be usable. For these reasons, it becomes possible to manufacture a surgical instrument having effective elasticity for so-called intestine forceps.

According to a ninth preferred aspect of the present invention, in the surgical instrument for operation purposes as defined in the seventh preferred aspect, (a) the treatment members are formed into scissors members having the shape of scissors, (b) the scissors members are pivotally supported on the treatment section, connected to the distal end of the insertion section, by way of a support shaft, (c) at least one or more scissors members relatively pivot around the support shaft between at least one close position and at least one open position, (d) the scissors members which are opposite to each other are formed in such a way that the distal ends cross each other when the scissors members situate at the open position, and (e) at least one or more scissors members is made by forging so that the intermediate portion has elasticity in such a direction in which the opposed scissors members push each other.

If a surgical instrument is formed in the shape of forceps, abrasion occurs as a result of rubbing action between blades, which may deteriorate the sharpness of the forceps. To prevent this, as disclosed in Japanese Patent Application No. Hei. 5-48245, the surface of forceps is covered with a titanium coating as a CVD coating to prevent the abrasion of forceps. Such a coating makes it possible to prevent the abrasion of the forceps, which, however, results in increased manufacturing cost. In the case where scissors members which can prevent abrasion but lack elasticity, as disclosed in U.S. Patent 5,234,453, if rubbing action is ensured in the scissors members to improve the sharpness of the scissors, an excessive force acts on a part which caulks the scissors. While the forceps are in use, the part caulking the scissors members deforms, and the rubbing area between the scissors becomes separated apart, thereby resulting in poor sharpness of the scissors.

In PCT WO 91/02493, a plate material is used as scissors members. This patent directed to ensure the sharpness of the scissors utilizing the elasticity of the plate material. However, since the distal ends of the scissors members are caulked, it was impossible to carry out minute incision, which is necessary in an operation, using the leading ends of the scissors members. Thus, the scissors of this type provided inconvenience in the practical use. In the case of the scissors members whose distal ends are not caulked, in some cases, the scissors members manufactured of a plate material become thin and lack mechanical strength, which may cause problems during minute operation of the scissors members.

The ninth aspect of the present invention is directed to solve problems in the prior art such as the abrasion of scissors members resulting from rubbing action, increased cost due to surface treatment to prevent abrasion, the difficulty of ensuring rubbing action of the scissors members because of the lack of elasticity, and the difficulty of carrying out minute cutting operation as a result of the manufacture of the scissors members from a plate material in order to maintain rubbing action of the scissors members.

By virtue of the configuration as defined in the ninth aspect of the present invention, scissors members used as the treatment members are manufactured by forging in such a way that a fiber flow appears in a longitudinal direction of the scissors members, whereby the surface hardness and abrasion resistance of the scissors members are improved without the need of special surface treatment. In this way, the sharpness of the scissors members is maintained. Further, the scissors members are provided with elasticity, and hence no excessive force acts on the part which caulks the opposed scissors members, which provides effective rubbing action and superior sharpness. Since the shape of the scissors members can be freely set, it is possible to manufacture scissors members into the shape suitable for minute operation when compared with the manufacture of scissors members using a plate material. In this way, it is possible to manufacture scissors members corresponding to the practical use of the scissors members.

According to a tenth preferred aspect of the present invention, in the surgical instrument for operation purposes as defined in either the seventh or eighth preferred aspect, (a) even after the treatment members have first come into contact with each other, the treatment member drive mechanism is driven in such a direction in which the treatment members are pushed in the direction of the close position, as a result of the deformation of the treatment members.

According to an eleventh preferred aspect of the present invention, in the surgical instrument for operation purposes as defined in any one of the seventh to ninth preferred aspects, (a) the operation means is provided with a rachet mechanism, (b) the treatments are pushed toward at least the first position, the second position, or the close position, (c) the amount of engagement of the rachet mechanism increases, and (d) after the treatment members have been pushed toward at least the first position, the second position, or the close position, (e) the treatment members are deformed, so that the amount of engagement of the rachet mechanism increases.

As disclosed in U.S. Patent 5,234,453, the treatment members are manufactured by machining or casting, and hence the treatment members lack elasticity. For this reason, when the treatment members are closed, when the treatment members clipped tissue, or when a certain amount of force is exerted on the treatment members as a result of grasping of tissue, the treatment member drive mechanism is not driven any further even if a further operating force is exerted on operation means. Therefore, the user feels as if the operation means is suddenly fixed not only during the course of the adjustment of the amount of a grasping force but also the moment at which the treatment members are closed or at which the tissue is grasped by the treatment members as the user pushes the operation means in the closing direction. In this way, the user is dissatisfied with terribly poor feeling of operation. This may sometimes injure user's hands and make the user fatigued.

In the case of an instrument for grasping purposes, operation means is provided with a rachet mechanism. However, in the previously mentioned state, the ratchets have already engaged with each other up to the first tooth, but stable grasping is not obtained in this state. If the user wants to further increment the engagement of the ratchets by one, such a further increase in the engagement of the ratchets was impossible for the conventional instrument, thereby resulting in unstable a grasping state.

On the other hand, in the case of surgical forceps as disclosed in U.S. Patent 3,101,715, treatment members and an operation member are made by forging, and hence appropriate deflection arise in these members. Even after the treatment members have been closed or even if a certain amount of force is exerted on the members as a result of the grasping of tissue, it is possible to move the operation means further and to drive the treatment members. Moreover, if the user wants to further increment the engagement of the ratchets by one while the treatment members are already in a grasping state as previously mentioned, it is possible to increase the engagement thanks to the elastic deflection of the treatment members and the operation member.

As disclosed in EPA-543,107 A2 or U.S. Patent 5,222,973, treatment members are manufactured from a plate material, and these patents attempted to solve the problems, that is, the previously mentioned poor feeling of operation and assurance of a continued stable grasping state by employing retractable type members and utilizing the elastic deformation of the treatment members. Since the treatment members are made of a plate material, and since the treatment members are retractable type, it was impossible for the treatment members to carry out separation action in the same manner as in the previously mentioned case. The instrument using these treatment members provided poor operability.

The tenth and eleventh aspects of the present invention are directed to solve the above mentioned drawbacks in the prior art. In other words, since it was impossible for the conventional treatment members to control a grasping force by deforming when the treatment members grasped tissue, the operation means was in a fixed state where the tissue was grasped, thereby resulting in terribly poor operability. This causes pains in user's hands or fatigue. In many cases, an inexperienced operator usually assists separation operation by grasping tissue. However, in such a case, it is extremely difficult for the inexperience operator to keep providing a constant grasping force, and therefore the operator may drop the tissue or causes damage to the tissue by an excessive grasping force. Further, in such a case, if the engagement between the ratchets is increased by one when it was desired to increase the engagement between the ratchets of the operation means by one to maintain the stable grasping state, an excessive force is exerted on the tissue. A conventional retractable treatment member drive mechanism or a conventional treatment member drive mechanism in which treatment members are formed from a plate material, both of which were conceived to solve the problems, could not carry out separation operation. Hence, the tenth and eleventh aspects of the present invention are intended to solve this problem.

By virtue of the configuration as defined in the tenth and eleventh aspects of this invention, treatment members are made by forging in such a way that a fiber flow appears in a longitudinal direction of the treatment members, as a result of this the treatment members are provided with resiliency. When the treatment members grasped the tissue, the treatment members deform. Hence, it becomes possible to drive the operation means up to a further elevated position. For example, if an operation force exerted on the operation means slightly changed while an inexperienced operator is operating the instrument, a stable grasping force will act on the tissue. Therefore, it becomes possible to reliably gras the tissue without damage. Moreover, if it is desired to increase the engagement between the ratchets by one to maintain a stable grasping state while the tissue is being grasped, it is possible to keep reliably holding the tissue intact without the application of an excessive force to the tissue even when the operation means is further moved in the closing direction from the state where the tissue is grasped.

According to a twelfth preferred aspect of the present invention, a surgical instrument for operation purposes comprises operation means for operating leading end treatment members, an insertion section, with one end thereof being connected to the operation means, having at the other end thereof a treatment section for treating tissue, and a treatment member drive mechanism for driving the treatment members in response to the operation of the operation means, wherein the treatment members or at least one of the members of the treatment member drive mechanism is manufactured by forging metallic materials.

By virtue of the configuration as defined in the twelfth aspect, at least one of the components which form the treatment members or the treatment member drive mechanism is made by forging metallic materials, and hence the productivity of constituent members of the treatment members and the treatment member drive mechanism is improved. As a result of this, it is possible to realize cost reduction, improved mechanical strength, reduced size and weight, assuarance of a degree of freedom of design, and facilitation of cleansing and sterilization of the inside of the insertion section.

### BRIEF DESCRIPTION OF THE DRAWINGS

Fig. 1 is a side elevation showing the diagrammatic structure of grasping type forceps which are a surgical instrument for operation purposes according to a first embodiment of the present invention;
Fig. 2A is a plan view showing a treatment section drive unit, and Fig. 2B is a side elevation of the same;
Fig. 3A is a partially cross-sectional plan view showing the principal configuration of the treatment section drive unit, and Fig. 3B is a side elevation of the same;

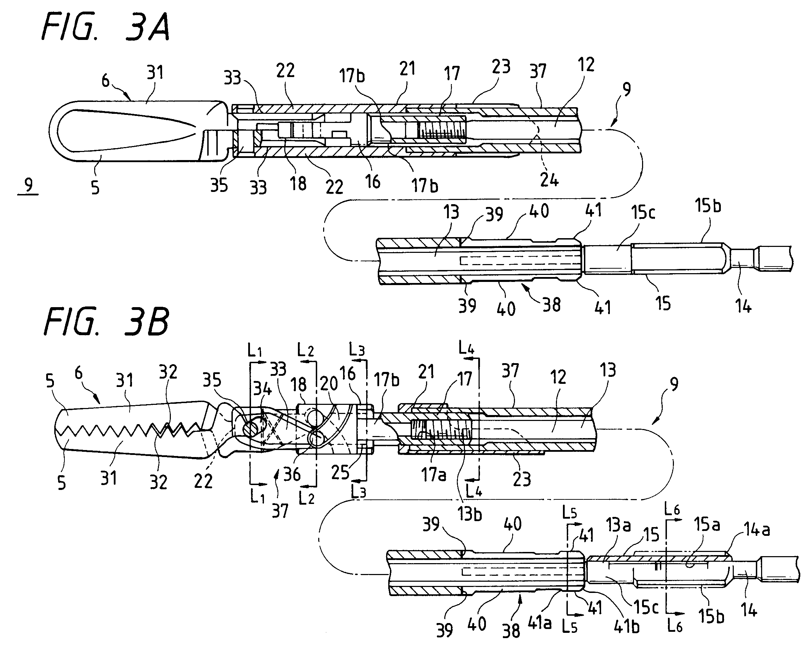

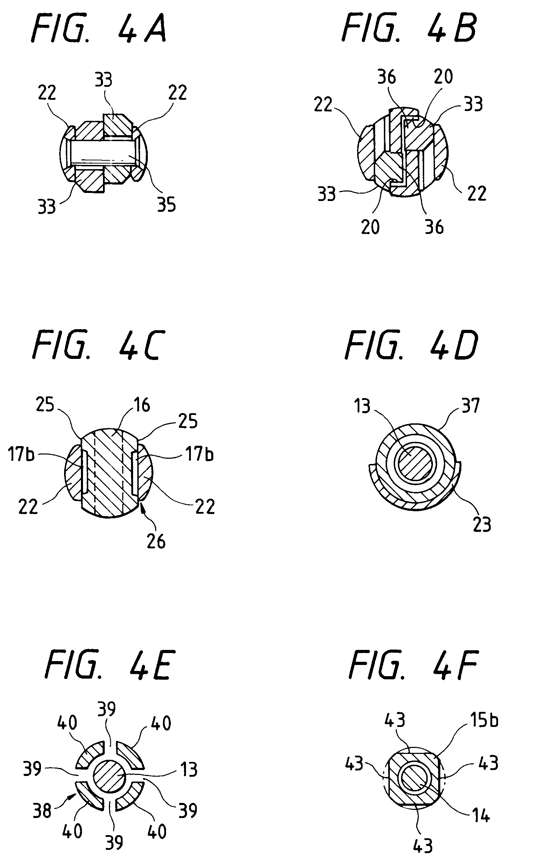

Fig. 4A is a cross-sectional view of the treatment section drive unit taken along line L₁ - L₁ shown in Fig. 3B, Fig. 4B is a cross-sectional view of the treatment section drive unit taken along line L₂ - L₂ shown in Fig. 3B, Fig. 4C is a cross-sectional view of the treatment section drive unit taken along line L₃ - L₃ shown in Fig. 3B, Fig. 4D is a cross-sectional view of the treatment section drive unit taken along line L₄ - L₄ shown in Fig. 3B, Fig. 4E is a cross-sectional view of the treatment section drive unit taken along line L₅ - L₅ shown in Fig. 3B, and Fig. 4F is a cross-sectional view of the treatment section drive unit taken along line L₆ - L₆ shown in Fig. 3B;
Fig. 5A is a partially cross-sectional plan view showing the leading end of the treatment section drive unit, Fig. 5B is a side elevation of the same, and Fig. 5C is a longitudinal cross-sectional view showing a snap fit arm of a connecting section of the treatment section drive unit;
Fig. 6A is a cross-sectional view of the treatment section drive unit taken along line L₁ - L₁ shown in Fig. 5B; and Fig. 6B is a transverse cross-sectional view showing a joint of a guide pin of a link arm used in a treatment member;
Fig. 7A is a longitudinal cross sectional view showing the leading end of a sheath unit, and Fig. 7B is a partially cross-sectional side elevation showing the rear end of the sheath unit;
Fig. 8A is a longitudinal cross-sectional view showing a positioning section of the leading end of a connecting pipe of the sheath unit, Fig. 8B is a longitudinal cross-sectional view of the sheath unit taken along line L₁ - L₁ shown in Fig. 8A, Fig. 8C is a longitudinal cross-sectional view showing a joint between the connecting pipe and an inner pipe of the sheath unit, Fig. 8D is a partially cross-sectional side elevation showing a snap fit of the rear end of the sheath unit, and Fig. 8E is a cross-sectional view of the sheath unit shown in Fig. 8D taken along line L₂ - L₂;
Fig. 9A is a partially cross-sectional side elevation showing an operating section, Fig. 9B is a front view showing a connection groove of a movable operation handle, and Fig. 9C is a cross-section of the operating section taken along line L₁ - L₁;
Fig. 10 is a longitudinal cross-sectional view showing the sheath unit when it is connected to an insertion section connecting section of a fixed handle of the operating section;
Fig. 11A is a longitudinal cross-sectional view showing the state in which a projection of the treatment section drive unit does not mesh with a projection of the sheath unit because they butt against each other, and Fig. 11B is a longitudinal cross-sectional view showing the state in which the movable operation handle is rotated until the handle comes into contact with a rubber cap while the projections are retained in the state as shown in Fig. 11A;
Fig. 12A is a longitudinal cross-sectional view showing the state in which the projection of the treatment section drive unit and the projection of the sheath unit are rotated to a proper fitting position, Fig. 12B is a longitudinal cross-sectional view showing the operating section which is in the state as shown in Fig. 12A;
Fig. 13A is a longitudinal cross-sectional view showing the projection when it fits into a storage section after having completely crossed an internal protuberance, and Fig. 13B is a longitudinal cross-sectional view showing the operating section which is in the state as shown in Fig. 13A;
Fig. 14A is a longitudinal cross-sectional view showing the grasping type forceps when inserted into a gap in tissue while a grasping member is closed, and Fig. 14B is a longitudinal cross-sectional view showing a connecting section between the treatment section drive unit and the sheath unit when they are in the state as shown in Fig. 14A;
Fig. 15A is a longitudinal cross-sectional view showing the grasping member when it is opened, and Fig. 15B is a longitudinal cross-sectional view showing the connecting section between the treatment section drive unit and the sheath unit when they are in the state as shown in Fig. 15A;

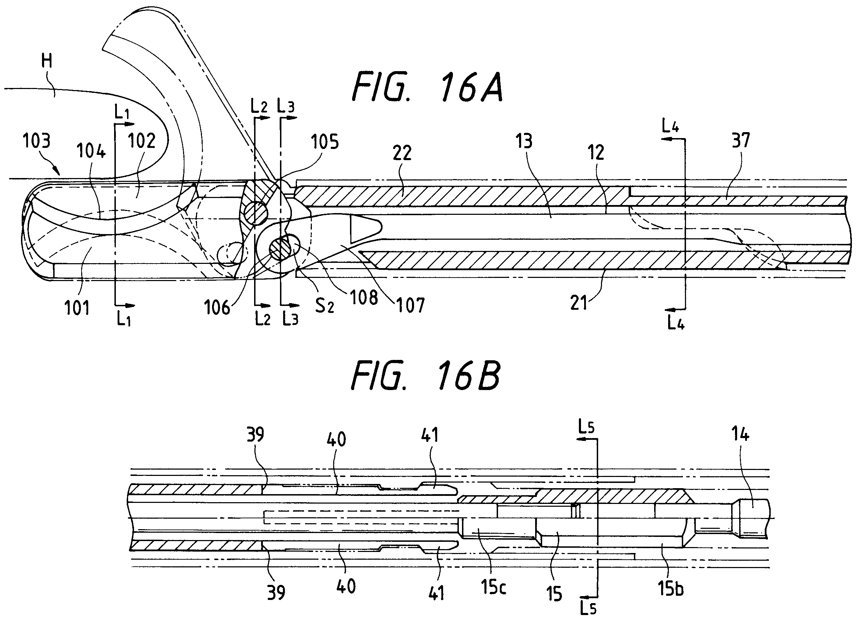

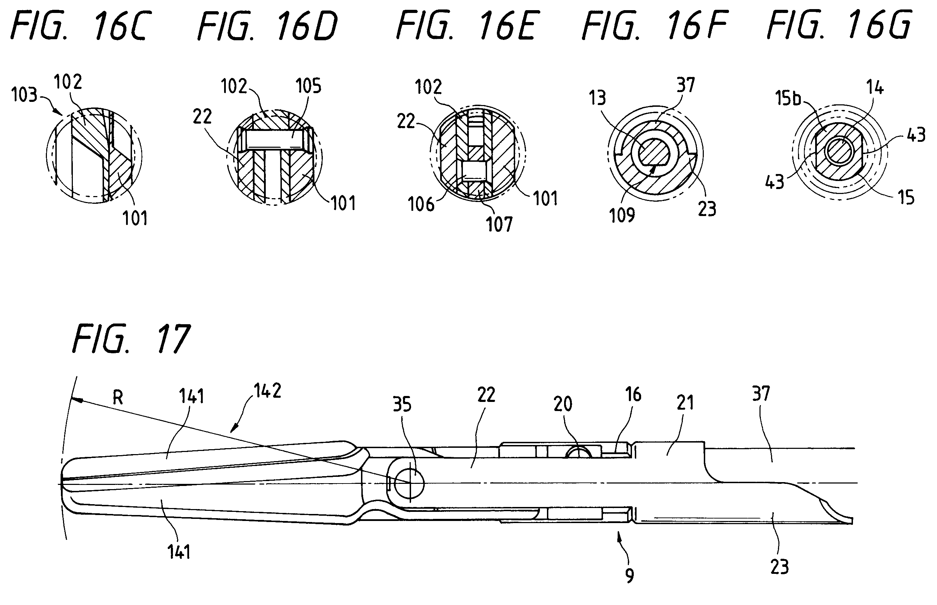

Figs. 16A - 16G schematically show a surgical instrument for operation purposes according to a second embodiment of the present invention, wherein Fig. 16A is a longitudinal cross-sectional view showing the diagrammatic structure of the leading end of grasp type forceps, Fig. 16B is a longitudinal cross-sectional view showing a connecting section between the treatment section drive unit and the sheath unit, Fig. 16C is a cross-sectional view of the forceps taken along line L₁ - L₁ shown in Fig. 3B, Fig. 16D is a cross-sectional view of the forceps taken along line L₂ - L₂ shown in Fig. 16A, Fig. 16E is a cross-sectional view of the forceps taken along line L₃ - L₃ shown in Fig. 16A, Fig. 16F is a cross-sectional view of the forceps taken along line L₄ - L₄ shown in Fig. 16A, and Fig. 16G is a cross-sectional view of the connecting section along line L₅ - L₅ shown in Fig. 16B;
Fig. 17 is a side elevation showing scissors type forceps according to a third embodiment of the present invention;
Figs. 18A and 18B show scissors type forceps according to a fourth embodiment of the present invention, wherein Fig. 18A is a longitudinal cross-sectional view showing the principal portion of the forceps, and Fig. 18B is a plan view showing a snap fit section;
Figs. 19A and 19B show forceps according to a fifth embodiment of the present invention, wherein Fig. 19A is a partially cross-sectional side elevation showing the leading end of a sheath unit, and Fig. 19B is a partially cross-sectional side elevation showing the handle-side end of the sheath unit;
Fig. 20A is a partially cross-sectional side elevation showing the diagrammatic construction of the leading end of a treatment section drive unit of the fifth embodiment, Fig. 20B is a longitudinal cross-sectional view showing a snap fit section of the treatment section drive unit, and Fig. 20C is a longitudinal cross-sectional view of the leading end of the treatment section drive unit taken along line L₃ - L₃ shown in Fig. 20A;
Fig. 21A is a cross-sectional view of the treatment section drive unit taken along line L₁ - L₁ shown in Fig. 20A, and Fig. 21B is a cross-sectional view of the treatment section drive unit taken along line L₂ - L₂ shown in Fig. 20A;
Figs. 22A to 22C show forceps according to a sixth embodiment of the present invention, wherein Fig. 22A is a partially cross-sectional side elevation showing the diagrammatic construction of the leading end of a treatment section drive unit, Fig. 22B is a longitudinal cross-section showing a snap fit section of the treatment section drive unit, and Fig. 22C is a cross-sectional view of the forceps taken along line L₃ - L₃ shown in Fig. 22A;
Fig. 23A is a cross-sectional view of the treatment section drive unit taken along line L₁ - L₁ shown in Fig. 22A, and Fig. 23B is a cross-sectional view of the treatment section drive unit taken along line L₂ - L₂ shown in Fig. 22A;
Figs. 24A and 24B show grasp type forceps according to a seventh embodiment of the present invention, wherein Fig. 24A is a longitudinal cross-sectional view showing the principal portion of the grasp type forceps when the forceps are opened, and Fig. 24B is a longitudinal cross-sectional view showing the principal portion of the forceps when the forceps are closed;
Figs. 25A - 25C show grasp type forceps according to an eighth embodiment of the present invention, wherein Fig. 25A is a side elevation of the overall forceps showing the opening and closing action of the forceps, Fig. 25B is a partially cross-sectional side elevation showing the principal portion of the forceps, and Fig. 25C is a side elevation of the principal portion showing the opening and closing action of the forceps;
Fig. 26A is a side elevation showing grasp type forceps according to a ninth embodiment of the present invention, and Fig. 26B is a side elevation showing grasp type forceps according to a tenth embodiment of the present invention;
Fig. 27 is a side elevation of grasp type forceps, according to an eleventh embodiment of the present invention, showing closing and opening action of the forceps;
Figs. 28A and 28B are a side elevation and a top view of grasp type forceps according to a twelfth embodiment of the present invention;
Figs. 29A to 29E are diagrammatic representations showing grasp type forceps according to a thirteenth embodiment of the present invention when the forceps are opened and closed;
Fig. 30 is a side elevation of a surgical instrument for operation purposes, which employs the grasp type forceps of the thirteenth embodiment of the present invention shown in Fig. 29, showing the opening and closing action of the surgical instrument;
Figs. 31A to 31C are partial cross-sectional views of grasp type forceps according to a fourteenth embodiment of the present invention showing variations in grasping states of a grasping member;
Fig. 32 is a diagrammatic representation showing a surgical instrument for operation purposes having another drive mechanism according to the present invention;
Fig. 33 is a diagrammatic representation showing a surgical instrument for operation purposes having still another drive mechanism according to the present invention;
Figs. 34A and 34B are partial side elevations of grasp type forceps according to the present invention showing opened and closed states of the forceps;
Figs. 35A and 35B are partial side elevations of grasp type forceps of another type according to the present invention, wherein Fig. 35A shows an opened state of the forceps, and Fig. 35B shows a closed state of the same;
Figs. 36A and 36B are partial side elevations of scissors type forceps according to the present invention, wherein Fig. 36A shows an opened state of the forceps, and Fig. 36B shows a closed state of the forceps;
Figs. 37A and 37B are partial side elevations showing a pushing-aside element according to the present invention, wherein Fig. 37A shows an opened state of the pushing-aside element, and Fig. 37B shows a closed state of the pushing-aside element;
Fig. 38 is a side elevation showing a surgical instrument for operation purposes according to the present invention wherein an insertion section is formed into a coil shape;
Fig. 39 is a side elevation showing a surgical instrument having a ratchet mechanism according to the present invention;
Fig. 40 is a side cross-sectional view showing a needle retainer according to the present invention; and
Figs. 41A and 41B are front cross-sectional views of the needle retainer shown in Fig. 40, wherein Fig. 41A shows an opened state of the needle retainer, and Fig. 41B shows a closed state of the needle retainer.

### DETAILED DESCRIPTION OF THE PREFERRED EMBODIMENTS

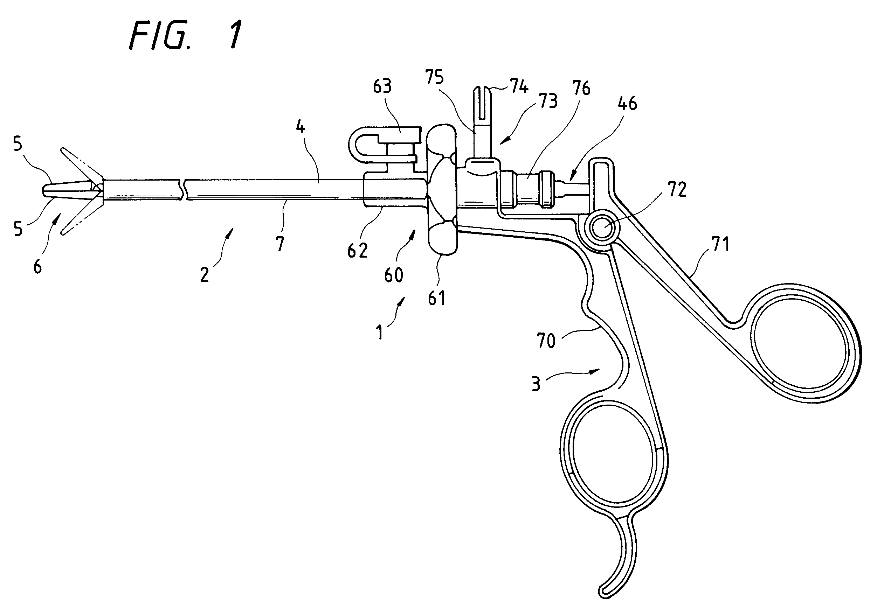

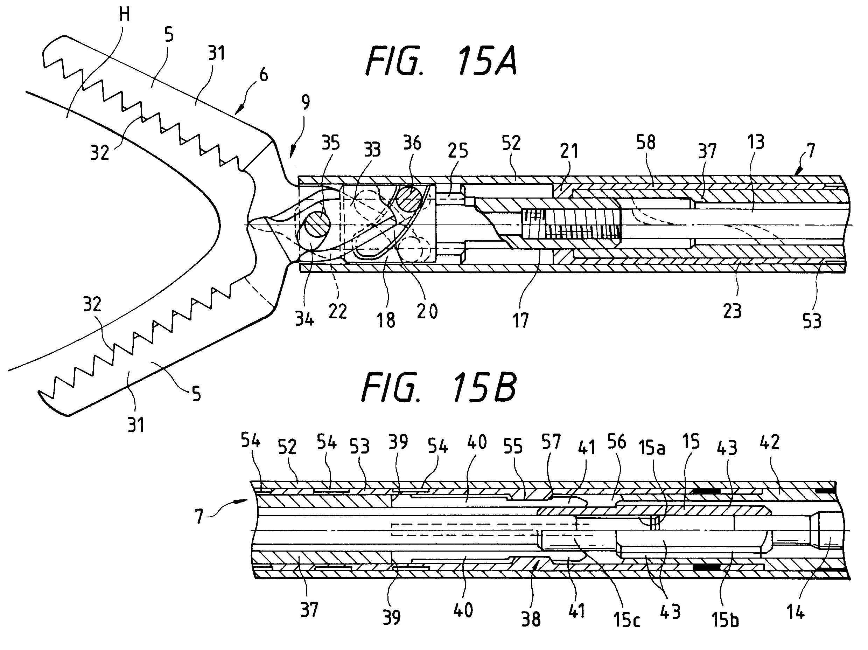

With reference to Figs. 1 to Figs. 15A and 15B, grasp type forceps according to a first embodiment of the present invention will be described. Fig. 1 diagrammatically shows the structure of grasp type forceps 1 which are used as a surgical instrument for operation purposes. In the forceps 1, an operation unit (operating means) 3 is connected to the handle-side base portion of an insertion portion 2 to be inserted into a living body through a tracheal mantle tube (not shown).

The insertion portion 2 has an extended-shaft-like shaft body 4. A treatment section 6 is provided at the leading end of this shaft body 4, and the treatment section 6 includes a pair of open/close grasping members (a treatment member) 5, 5. The shaft body 4 of the insertion section 2 is covered with a sheath unit 7 which forms an outer shell of the insertion section 2. The grasping members 5, 5 are manufactured by forging metallic materials, for example, stainless steel, titanium, and aluminum.

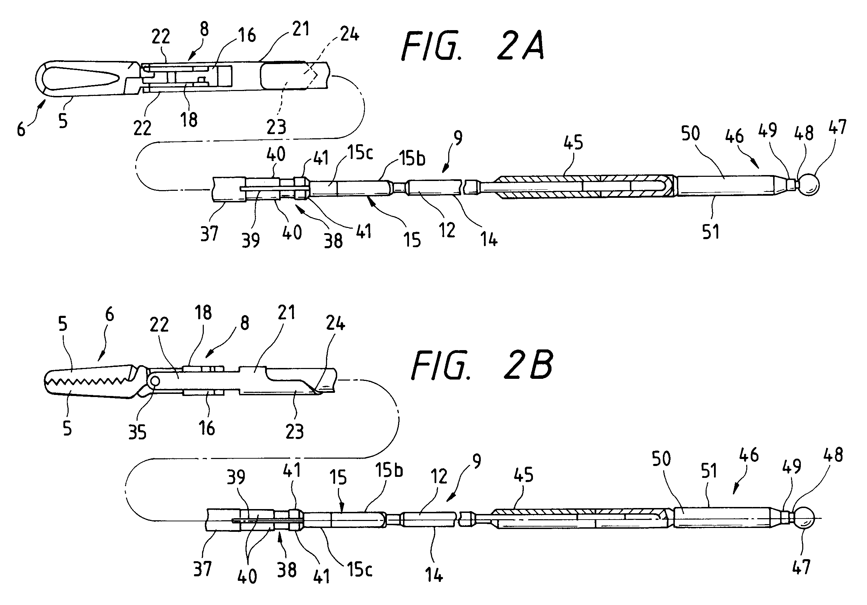

Further, a treatment section drive unit (a treatment member driving mechanism) 9 is disposed within the sheath unit 7 as shown in Figs. 2A and 2B, and the treatment section drive unit 9 is integrated with an open/close drive mechanism 8 for opening and closing the grasping members 5, 5 of the treatment section 6.

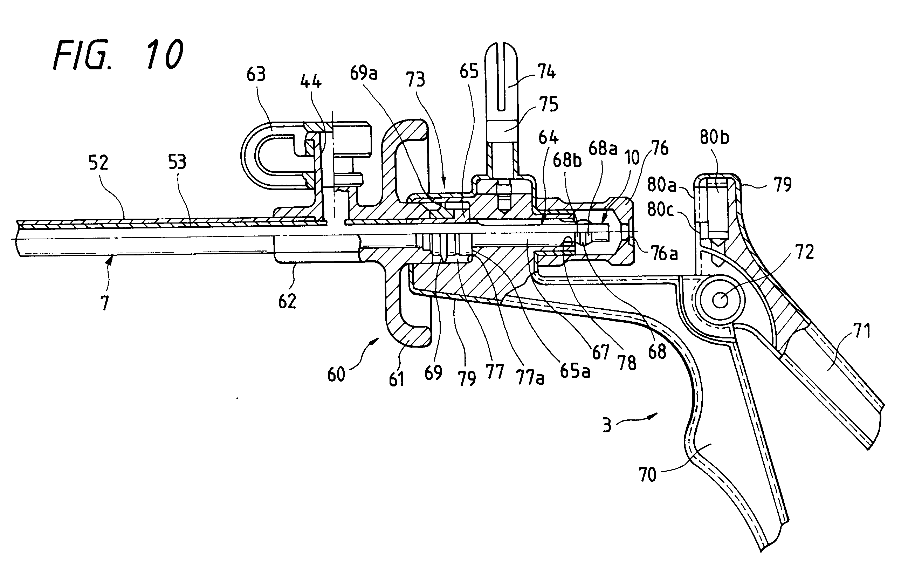

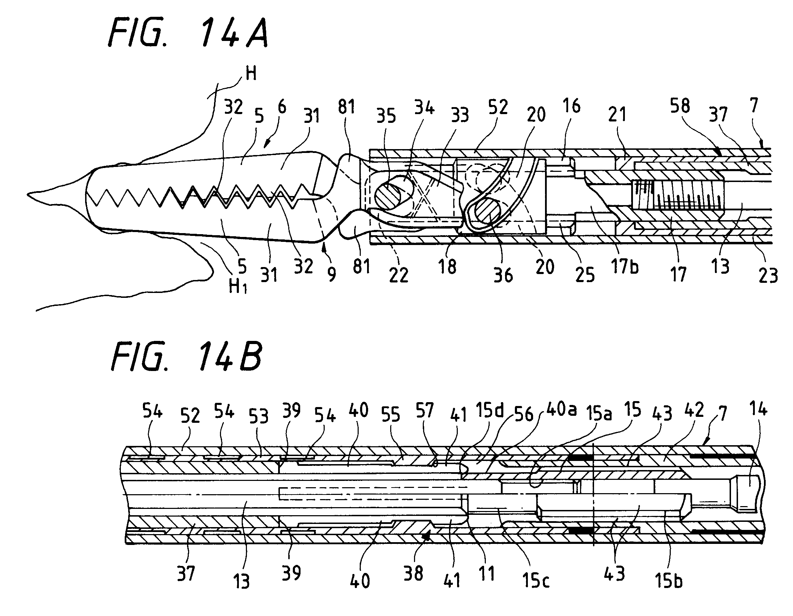

The main body of the grasp type forceps 1 is made up of three independent units which are assembled separately from each other; namely, the treatment section drive unit 9, the sheath unit 7, and the operation unit 3. The operation unit 3 and the sheath unit 7 are removably connected with each other via a first connecting section 10 (shown in Fig. 10) which will be described later, whereas the sheath unit 7 and the treatment section drive unit 9 are also removably connected with each other via a second connecting section 11 (shown in Fig. 14B) which will be described later.

The treatment section drive unit 9 is provided with an actuation shaft 12 for actuating the grasping members 5, 5. This actuation shaft 12 is disposed within the sheath unit 7 in such a way that it can advance or recede in the sheath unit. Further, as shown in Figs. 3A and 3B, the actuation shaft 12 is provided with a leading-end side rod-shaped operation rod 13, a handle-side operation rod 14 disposed close to the handle of the surgical instrument, and a cylindrical operation rod connecting member 15 for connecting together the leading-end side operation rod 13 and the handle-side operation rod 14. These leading-end side operation rod 13 and the handle-side operation rod 14 are manufactured by forging metallic materials such as stainless steel.

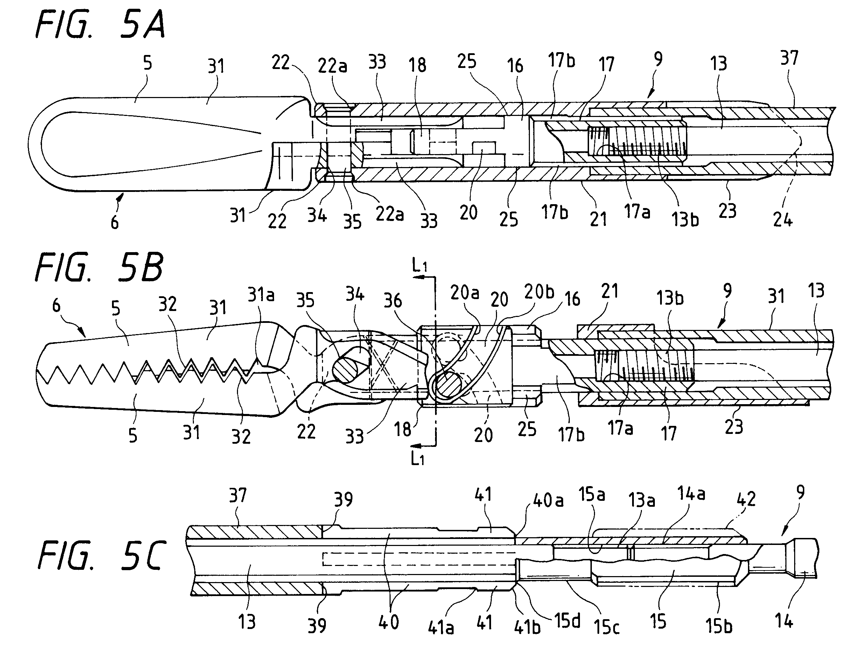

A thread 15a is cut on the internal peripheral surface of the operation rod connecting member 15 as shown in Fig. 5C. An external thread 13a and an external thread 14a which are screwed into the thread 15a of the operation rod connecting member 15 are respectively formed on the rear-end outer peripheral surface of the leading-end side operation rod 13 and the leading-end outer peripheral surface of the handle-side operation rod 14. When the external thread 13a of the leading-end side operation rod 13 and the external thread 14a of the handle-side operation rod 14 are screwed into the thread 15a of the operation rod connecting member 15, the three members, that is, the leading-end side operation rod, the handle-side operation rod, and the operation rod connecting member are assembled into one.

A forceps opening and closing member 16 is connected to the leading end of the leading-end side operation rod 13, as shown in Figs. 5A and 5B. This forceps opening and closing member 16 is manufactured by forging metallic materials such as stainless steel. A cylindrical operation rod connecting section 17 is formed at the rear end of the forceps opening and closing member 16. An internal thread 17a is cut on the internal surface of this operation rod connecting section 17, and an external thread 13b formed on the leading-end outer peripheral surface of the leading-end side operation rod 13 is screwed into the internal thread 17a. Water flow channels 17b are formed in the outer peripheral surface on both sides of the operation rod connecting section 17 of the forceps opening and closing member 16 over the entire length of the operation rod connecting section 17, as shown in Figs. 3A and 4C. Heretofore, if such a space for cleansing the instrument is ensured, the cross-sectional area of the operation rod connecting section 17 is reduced, which in turn results in insufficient mechanical strength of the operation rod connecting section. To compensate for the insufficient mechanical strength, the connecting section needs to be additionally quenched after having been machined, which adds to the manufacturing cost. However, the forceps opening and closing member 16 of the grasp type forceps 1 of the present invention is manufactured by forging metallic materials, and therefore required mechanical strength is ensured without the need of the above mentioned additional processing.

A substantially rectangular plate-like grasp member connecting section 18 is provided at the leading end of the forceps opening and closing member 16. Approximately circular-arc pin slide slots 20 are respectively formed in both sides of this grasp member connecting section 18, as shown in Fig. 5B.

A leading end cover 21 for guiding the movement of the actuation shaft 12 in the direction of the axis is provided around the forceps opening and closing member 16. This leading end cover 21 is manufactured by forging metallic material such as stainless steel. A caulking pin 35, which will be described later, is caulked at the leading end cover 21 in such a way that the grasping members 5, 5 are actuated smoothly. Therefore, stress develops around the caulking pin 35. Force for grasping and releasing action is transmitted to the caulking pin 35 through elongated holes 34, as a result of which strong stress exerts on support arms 22 of the leading end cover 21. In the case of the grasp type forceps 1 of this invention, the leading end cover 21 is manufactured not by machining but by forging. Therefore, as previously mentioned, the leading end cover is less likely to fracture and safer when compared with a conventional leading end cover manufactured by machining.

Further, the two support arms 22, 22 project from the leading end of the leading end cover 21. A projection 23 for positioning in a rotational direction projects from the rear end of the leading end cover 21, and this projection 23 has an approximately semicircular shape as shown in Fig. 4D. A cut-away portion 24, which is cut substantially at an angle of 90 degrees as shown in Fig. 2A, is formed at the handle-side end of the projection 23. Planar notches 25, 25, which come into contact with the two support arms 22, 22 of the leading end cover 21, are formed between the grasp member connecting section 18 and the operation rod connecting section 17 of the forceps opening and closing member 16, as shown in Fig. 4C. An interval between the notches 25, 25 is set equal to an interval between the two support arms 22, 22 of the leading end cover 21. A rotation prevention section 26 is formed by contact between the notches 25, 25 of the forceps opening and closing member 16 and the two support arms 22, 22 of the leading end cover 21. This rotation prevention section 26 prevents the rotation of the forceps opening and closing member 16 in relation to the center axis thereof.

Each of the grasping members 5 of the treatment section 6 is provided with a grasp section 31 for grasping tissue H. Forceps teeth 32 approximately shaped like a sawtooth are formed respectively along mating faces of the grasp sections 31 of the grasping members 5. Conventionally, these forceps teeth 32 are formed by machining, and hence it is difficult to obtain a cost reduction effect resulting from the mass-production of surgical instruments, thereby leading to an increased cost. However, in the case of the grasp type forceps of this invention, the grasping members 5 are manufactured by forging metallic material. Therefore, the forceps teeth 32 can be formed at one time using molds, thereby resulting in cost reduction.

For example, letters or symbols denoting a product name or a company name are inscribed on the back plate of the grasp sections 31 of the grasping members 5 by means of laser marking means. A gap 31a is opened in a handle-side portion of a contact area between the grasp sections 31, 31 of the grasping members 5, 5 formed when the grasping members 5, 5 are closed, whereby tissue H can be reliably grasped.

Further, a link arm 33 projects from the base end of each of the grasping member 5. An elongated hole 34 is formed in the link arm 33 at some midpoint therein, as shown in Fig. 5B. The caulking pin 35 which becomes a pivot is inserted into the elongated holes 34 of the link arms 33 through pin insertion holes 22a formed in the leading ends of the two support arms 22 of the leading end cover 21, as shown in Fig. 5A. Both ends of this caulking pin 35 are caulked and fixed to the two support arms 22, 22. Both of the grasping members 5 of the treatment section 6 are supported by the support arms 22, 22 of the leading end cover 21 in such a way as to be pivotable on the caulking pin 35. In this case, a cam mechanism is formed by combination of the caulking pin 35, which serves as the center of the opening and closing action of the grasping members 5, 5, with the elongated holes 34 of the grasping members 5, 5.

A guide pin 36 projects from the leading end of the link arm 33 of each of the grasping members 5, 5. This guide pin 36 is inserted into the pin slide slit 20 of the forceps opening and closing member 16 in such a way as to be slidable. If the actuation axis 12 is moved in the axial direction with respect to the leading end cover 21, the forceps opening and closing member 16 moves in the axial direction together with the actuation axis 12. Association with the movement of the forceps opening and closing member 16, the guide pins 36 move along the pin slide slots 20, so that the link arms 33 of the grasping members 5, 5 of the treatment section 6 pivot on the caulking pin 35. As a result of this, the grasp sections 31 of the treatment section 6 are actuated between a closed position designated by a solid line and an opened position designated by a phantom line, both being shown in Fig. 1, whereby the open/close drive mechanism 8 is formed. The grasping members 5 of the treatment section 6 are opened when the actuation shaft 12 is pushed, whilst the grasping members 5 are closed when the actuation shaft 12 is pulled. Conventionally, these grasping members 5, 5 were manufactured by machining or casting. Particularly when the grasping members were manufactured by machining, sufficient mechanical strength of the grasping members for a required shape was not obtained because of restrictions in size. To compensate for the insufficient mechanical strength, the grasping members are additionally quenched or subjected to another treatment so as to increase the mechanical strength of the surgical instrument. This made it impossible to avoid a rise in manufacturing costs. However, in the case of the forceps 1 of this invention, the grasping members 5, 5 are manufactured by forging metallic material. Sufficient and necessary mechanical strength are obtained when the grasping members are molded, which makes additional quenching unnecessary and hence brings the manufacturing cost in line.

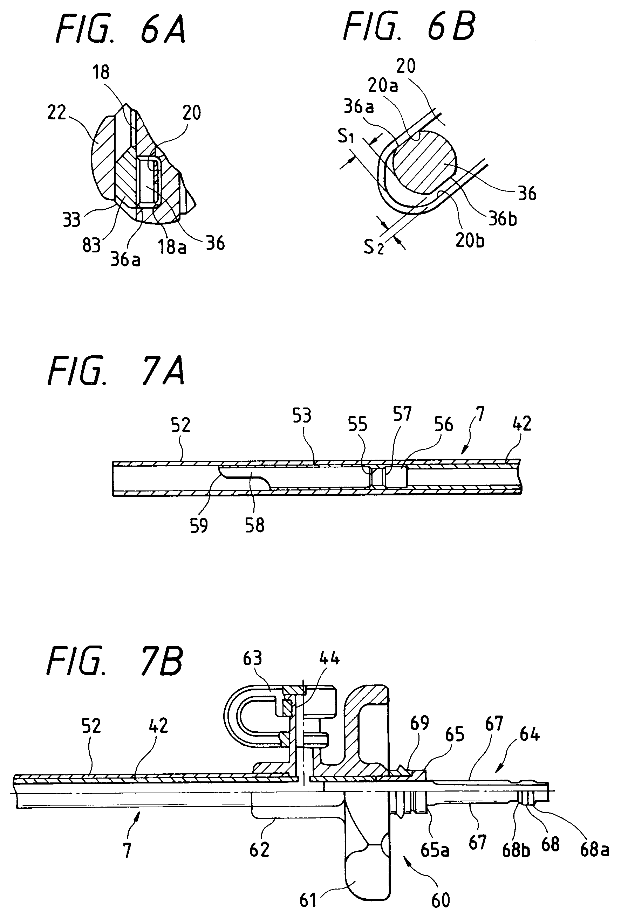

A clearance S₁ is formed in the pin slide slot 20 of the grasp member connecting section 18 of the forceps opening and closing member 16, and the clearance S₁ has a required width between the position of the guide pin 36 when the grasping members 5, 5 are closed and the end of the pin slide groove toward the leading end of the forceps, as shown in Fig. 6B. Further, a backlash S₂ having a required width is formed in the pin slide groove 20 between the guide pin 36 and a handle-side slot side surface 20b of the pin slide slot 20. This backlash S₂ is formed in the pin slide slot 20 when the guide pin 36 of the link arm 33 is brought into contact with a leading-end-side slot side surface 20a of the pin slide slot 20 as a result of the closing action of the grasping members 5, 5.

The guide pin 36 of each of the connecting members 5, 5 has a handle-side contact surface 36b and a leading-end-side contact surface 36a, as shown in Fig. 6B. The contact surface 36b is brought into slidable contact with the slot side surface 20b of the pin slide slot 20 when the forceps opening and closing member 16 is pressed toward the leading end of the forceps when the grasping members 5, 5 are opened. The contact surface 36a is brought into slidable contact with the slot side surface 20a of the pin slide slot 20 when the forceps opening and closing member 16 is pulled to the handle side when the grasping members 5, 5 are closed. The curvature radius of the contact surface 36a of the guide pin 36 is set substantially equal to the curvature radius of the slot side surface 20a of the pin slide slot 20. Similarly, the curvature radius of the contact surface 36b is set substantially equal to the curvature radius of the slot side surface 20b of the pin slide slot 20.

As shown in Fig. 6A, fillets 36c are formed at the root of the guide pin 36, and fillets 18a are also provided between the bottom surface and the side surfaces of the pin slide slot 20 of the grasp member connecting section 18 in the forceps opening and closing member 16. By virtue of these fillets, the guide pin 36 and the pin slide slot 20 are prevented from being cracked from the above mentioned points. Moreover, a minimum cross-sectional area section 83 is provided at a leading-end-side area around the root of the guide pin 36 of the link arm 33 of each grasping member 5. This minimum cross-sectional area section 83 has the minimum cross sectional area compared with other portions of the grasping member 5.

The leading end of a connection pipe 37 is fixedly fitted to the inside of the semicircular projection 23 provided at the rear end of the leading end cover 21, as shown in Fig. 4D. The leading end cover 21 and the connection pipe 37 are fixedly connected together by bonding means, soldering means, brazing means, welding means, or the like. They may be integrated into one unit by molding.

A snap fit section (a connection section) 38 which is removably connected to the sheath unit 7 is provided at the rear end of the connection pipe 37. As shown in Fig. 4E, cantilever-beam-shaped four snap fit arms (an arm section) 40 are formed in the snap fit section 38 by cutting four slits 39 in the rear end side of the snap fit section 38 close to the connection pipe 37. Stainless steel SUS304T is ordinarily used for the connection pipe 37. However, stainless steel SUS 420J2 may be alternatively used to improve the abrasion resistance of the connection pipe 37. As a matter of course, the snap fit section 38 may be formed by forging. Protuberances (engagement protuberances) 41 are raised from the outer peripheral surface at the opposite end of each of the snap fit arms 40 of the snap fit section 38 in relation to the connection pipe 37. A leading-end-side slope 41a is formed by tapering the leading-end-side end face of the protuberance 41, whereas a handle-side slope 41b is also formed by tapering the rear-end-side end face of the protuberance 41.

The rear end of the leading-end-side operation rod 13 of the actuation axis 12 projects from the snap fit section 38 of the leading end cover 21 in the direction of the handle. This rear end is screwed into the operation rod connection member 15 outside the snap fit section 38.

A centering section 15b having a large diameter is formed at the handle-side end of the outer peripheral surface of the operation rod connection member 15. The outer diameter of this centering section 15b is set substantially equal to the inner diameter of an inner pipe 42 of the sheath unit 7 which will be described later. Further, four water guide notches 43 are formed along the outer peripheral surface of the centering section 15b, as shown in Fig. 4F. When the treatment section drive unit 9 is attached to the sheath unit 7, and when the centering section 15b of the operation rod connection member 15 is inserted into an inner pipe 42 of the sheath unit 7, as shown in Fig. 14B, water or the like poured from a water filling cock 44, which will be described later, flows into a space between the notches 43 of the centering section 15b and the internal peripheral surface of the inner pipe 42 of the sheath unit 7. So long as the flow of water through the inside of the inner pipe 42 is ensured, the number of notches 43 formed in the outer peripheral surface of the centering section 15b is not limited to four but may be set to any desired value.

A leading end small diameter section 15c whose diameter is substantially the same as the inner diameter of the connection pipe 37 is formed at a leading-end-side area of the outer peripheral surface of the operation rod connection member 15 with respect to the centering section 15b. Together with the advancing and receding action of the actuation shaft 12 occurring when the grasp section 31 of the treatment section 16 is opened and closed, the leading end small diameter section 15c can enter the inside of the snap fit arms 40 of the connection pipe 37. A tapered section 15d is formed on the leading-end-side end face of the leading end small diameter section 15c of the operation rod connection member 15. A tapered section 40a corresponding to the tapered section 15d of the operation rod connection member 15 is formed on the inside of the handle-side end face of the projection 41 of each of the snap fit arms 40.

The rear end of the handle-side operation rod 14 is fixedly screwed into a pipe cover 45 and a connecting member 46 which are made of resin such as polysulfone. A spherical connection ball 47 is connected to the end of the connection member 46 facing the operation section unit 3 in an engageable and disengageable manner. This connection ball 47 is made by forging stainless steel.

Further, this connection member 46 has a small diameter section 48 connected to the leading-end-side of the connection ball 47, and a large diameter section 50 connected to the leading-end-side of the small diameter section 48 via a middle diameter section 49 having an intermediate diameter. The outer peripheral surface of the connection member 46 is coated with a heat-shrinkable tube 51 made of Teflon to ensure electrical insulation of the connection member.

Figs. 7A and 7B show the sheath unit 7. The outer peripheral surface of the sheath unit 7 is covered with an insulating tube 52, and the metallic inner pipe 42 made of stainless steel or the like is provided inside of the insulating tube 52.

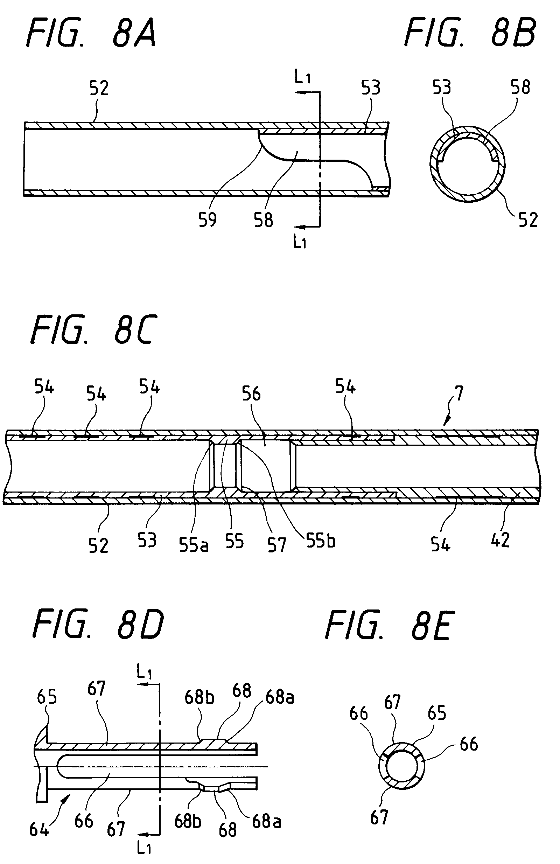

A rear end portion of a connection pipe 53 for connecting the treatment section drive unit 9 with the sheath unit 7 is connected to the leading end portion of the inner pipe 42 by means of brazing means or laser-welding means, as shown in Fig. 7A. A plurality of adhesive flutes 54 for storing an adhesive are formed in the outer peripheral surface of the connection pipe 53, as shown in Fig. 8C. similarly, the adhesive flutes 54 are formed in the outer peripheral surface of the leading end portion of the inner pipe 42. The insulating tube 52 and the connection pipe 53, and the insulating tube 52 and the inner pipe 42 are respectively bonded together by means of an adhesive applied to the inside of the adhesive flutes 54.

An internal protuberance 55 is radially raised from the inner peripheral surface of the rear end portion of the connection pipe 53 toward the center axis. The diameter of this internal protuberance 55 is smaller than the outer diameter of the snap fit arms 40 of the treatment section drive unit 9. Further, a holding section 56 is formed at an area of the inside of the connection pipe 53 closer to the handle with respect to the inner protuberance 55. This holding section 56 is intended to hold the protuberances 41 of the snap fit arms 40 which went over the internal protuberance 55 when the connection pipe 53 was connected to the treatment section drive unit 9. A step 57 which engages with, and disengages from, the protuberances 41 of the snap fit arms 40 of the treatment section drive unit 9 is formed between the holding section 56 and the inner protuberance 55. A tapered leading-end-side slop 55a is formed at the leading-end-side end face of the internal protuberance 55, whereas a tapered handle-side slope 55b is formed at the rear-end-side end face of the internal protuberance 55.

A positioning projection 58 for positioning the treatment section drive unit 9 projects from the leading end portion of the connection pipe 53. This positioning projection 58 is cut into a semicircular shape so as to mesh with the semicircular projection 23 of the leading end cover 21 of the treatment section drive unit 9, as shown in Figs. 8A and 8B. A tapered portion 59 with its leading end being rounded is formed in the leading-end-side edge of the positioning projection 58.

As shown in Fig. 7B, a joint section 60 which is removably connected to the operation section unit 3 is provided at the base portion of the sheath unit 7. This joint section 60 is provided with a rotary control 61 and the water filling cock 44.

A cylindrical body 62 is fitted around the base portions of the insulating tube 52 and the inner pipe 42 of the sheath unit 7. The rotary control 61 and the water filling cock 44 which are integrated into one body project from the outer peripheral surface of the cylindrical body 62. The water filling cock 44 is provided with a rubber stopper 63 for closing the water filling cock 44 in such a way that it can be opened or closed.

A snap fit section (a connection section) 64 which is removably connected to the operation section unit 3 is provided at the base portion of the joint section 60. This snap fit section 64 is provided with a connection pipe 65 made of stainless steel or the like. The leading end of this connection pipe 65 is fixedly inserted into the inside of the cylindrical body 62 from the base portion thereof, as shown in Fig. 7B.

Two snap fit arms (arm sections) 67 are formed in the rear end portion of the connection pipe 65 by cutting two slits 66 in the rear end portion as shown in Figs. 8D and 8E. As a matter of course, the snap fit sections 67 may be formed by forging. A protuberance (an engaging protuberance) 68 is formed on the outer peripheral surface of the leading end portion of each snap fit arm 67 of the snap fit section 64. This protuberance 68 has a first slope 68a formed on the side of the protuberance 68 facing the leading end of the snap fit arm 67 (on the handle side of the sheath unit), and a second slop 68b formed on the side of the protuberance 68 facing the root side of each snap fit arm 67 (on the leading end side of the sheath unit 67).

A butting surface 65a is formed at the root of each snap fit arm 67 in the rear end portion of the connection pipe 65. When the connection pipe 65 is connected to the operation section unit 3, the butting surface 65a butts against a butting surface 77a formed between a large diameter section 77 and a small diameter section 78 in an insertion section joint section 73 of a fixed handle 79 which will be described later. A ring-shaped rubber packing 69 is fitted around the outer peripheral surface of the portion of the connection pipe 65 of the snap fit section 64 connected to the cylindrical body 62.

In this embodiment, the first slope 68a of the protuberance 68 of each snap fit arm 67 is tapered at an angle θ₁ of about 30 degrees with respect to the center axis, whilst the second slope 68b is tapered at an angle θ₂ of about 45 degrees with respect to the center axis. However, these angles can be arbitrarily determined. For example, if the cone angle θ₁ of the first slope 68a is set smaller, the snap fit arms 67, 67 can be fit into the operation section unit 3 with a smaller force. Contrary to this, if the cone angle θ₁ of the first slope 68a is set larger, a larger force becomes needed to fit the snap fit arms into the operation section unit 3.

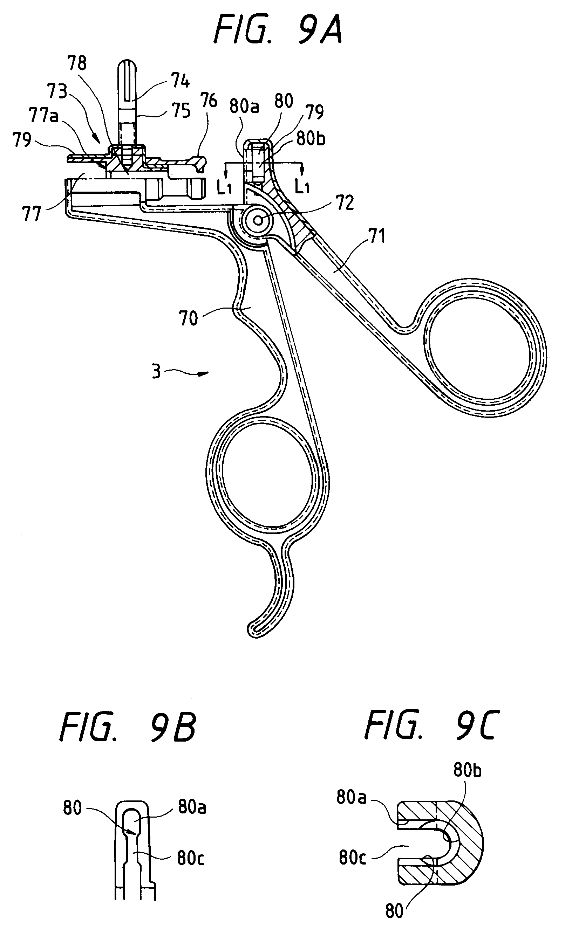

Fig. 9A shows the operation section unit 3 for opening and closing the grasping sections 5, 5 of the treatment section 6 which are disposed on the leading end side of the insertion section 2. This operation section unit 3 is provided with the approximately L-shaped fixed handle 70 and a movable operation handle 71. The movable operation handle 71 is pivotally connected to an L-shaped angled portion of the fixed handle 70 via a joint screw 72.

The tubular insertion section joint section 73 is provided above the fixed handle 70. An electrode pin 74, to which a connector of a power transmission code from a high-frequency power supply (not shown) is attached, projects from the top of the outer peripheral surface of the insertion section joint section 73. The outer peripheral surface of a lower portion of the electrode pin 74 is covered with an insulating pipe 75 made of insulating materials such as plastics. Letters or symbols denoting, for example, a product name or a company name are inscribed on the outer peripheral surface of this insulating pipe 75 by machining or molding. A rubber cap 76 is fitted into the rear end portion of the insertion section joint section 73 to ensure the waterproofness and airtightness of the sheath unit 7. An aperture 76a for permitting the insertion and removal of the connection ball 47 of the treatment section drive unit 9 is formed in the rubber cap 76.

The large diameter section 77 for holding the rubber packing 69 of the sheath unit 7 is formed on the leading-end side of the inside of the insertion section joint section 73, and the small diameter section 78 for holding the snap fit section 64 is formed being the large diameter section 77. The diameter of this small diameter section 78 is substantially the same as the outer diameter of the snap fit arms 67, 67 of the snap fit section 64, and the snap fit arms 67, 67 fit into the small diameter section 78.

The fixed handle 70 and the movable operation handle 71 are made of metallic materials by, for example, precision casting such as the lost wax casting method or the metal injection molding method. The exterior surfaces of the fixed handle 70 and the movable operation handle 71 are coated with an insulating coating film 79 such as fluorocarbon resin to ensure electrical insulation of them. The fixed handle 70 and the movable operation handle 71 may be molded of electrical insulating materials such as synthetic resins. If they are made of electrically nonconducting material, the insulating coating film 79 may be omitted. Moreover, the fixed handle 70 and the movable operation handle 71 may be formed by forging.

A joint groove 80 is formed in the vicinity of the upper end portion of the movable operation handle 71, and the connection ball 47 of the connection member 46 of the treatment section drive unit 9 is inserted into this joint groove 80. An entrance aperture 80a which has the same diameter as the connection ball 47 is formed in upper portion of the joint groove 80. Further, a ball guide hole 80b is vertically drilled in the inner part of the joint groove 80, and this ball guide hole 80b downwardly guides the connection ball 47 of the connection member 46.

A connection member guide section 80c is formed along both sides of the opening of the ball guide hole 80b around a lower part of the entrance aperture 80a, as shown in Figs. 9B and 9C. This connection member guide section 80c has a width smaller than the diameter of the connection ball 47 of the connection member 46 but larger than the width of the middle diameter section 49.

The entrance aperture 80a of the joint groove 80 into which the connection ball 47 of the connection member 46 is inserted is formed not in the upper end surface of the movable operation handle 71 but in the side surface on the leading end side of the joint groove 80. By virtue of this configuration, when various treatment is performed applying a high-frequency current to the grasp type forceps 1 of this embodiment, it becomes impossible for fingers of an operator to get in contact with the entrance aperture 80a. Like a conventional movable operation handle, if the entrance aperture 80a is opened in the upper end surface of the movable operation handle 71 with which fingers of the operator are likely to come into contact during treatment, the fingers of the operator come in contact with the opening formed in the upper end surface of the movable operation handle 71, and the operator burns his/her fingers by a high-frequency current leaked as a result of the touch of the fingers with the opening. However, as previously mentioned, the present invention can prevent such an accidental injury. Moreover, the ball guide hole 80b is drilled to the vicinity of the join screw 72, and hence the joint groove 80 can be much better cleansed compared with the conventional forceps.

The operation of the above construction of the forceps will be described hereinbelow. An explanation will be given of the assembly of the forceps 1 from the operation section unit 3, the sheath unit 7, and the treatment section drive unit 9 which are the three constituent elements of the surgical instrument main body and are in a disassembled state.

To being with, the operation section unit 3 of the grasp type forceps 1 and the sheath unit 7 which forms the outer shell of the insertion section 2 are connected together. When the operation section unit 3 is connected to the sheath unit 7, the snap fit section 64 of the sheath-unit 7 is inserted into the small diameter section 78 through the large diameter section 77 opened on the leading side of the insertion section joint section 73 of the fixed handle 70. The outer diameter of the snap fit arms 67, 67 of the snap fit section 64 is substantially the same as the inner diameter of the small diameter section 78, and therefore the snap fit arms 67, 67 fit into the small diameter section 78. The outer diameter of the protuberance 68 is set larger than that of the small diameter section 78. For this reason, when the protuberances 68 formed at the leading end of each of the snap fit arms 67, 67 are inserted into the small diameter section 78, the snap fit arms 67, 67 are resiliently deformed and deflect toward the center axis.

In this way, while the snap fit arms 67, 67 are bent to the center axis, the sheath unit 7 is further inserted into the inside of the small diameter section 78 of the insertion section joint section 73 of the fixed handle 70. When the butting surface 65a formed at the rear end portion of the connection pipe 65 of the sheath unit 7 butts against the butting surface 77a between the large diameter section 77 and the small diameter section 78 in the insertion section joint section 73 of the fixed handle 70, the protuberances 68 at the leading ends of the snap fit arms 67, 67 spread out from the small diameter section 78. At this time, the slope start point of the second slope 68b of the protuberance 68 of each snap fit arm 67 substantially comes into contact with the handle-side end of the small diameter section 78. The snap fit arms 67, 67 spread from their indwardly bent states to their original states by means of the resilient form of the snap fit arms, whereby the snap fit arms resume their original shapes.

In this way, when the snap fit arms 67, 67 resume their original shapes, the protuberance 68 of each of the snap fit arms 67 removably engages with the rear end surface of the insertion section joint section 73 of the fixed handle 70, as shown in Fig. 10. As a result of this, the sheath unit 7 is connected to the insertion section joint section 73 of the fixed handle 70 of the operation section unit 3. At this time, the snap fit section 64 will not disengage from the small diameter section 78 of the insertion section joint section 73 unless the protuberances 68 deflect to the inner diameter of the small diameter section 78 of the insertion section joint section 73 within the range where the resilient force of the snap fit arms 67, 67 is effective.

While the sheath unit 7 is connected to the operation section unit 3, the sheath unit 7 is supported by the operation section unit 3 in such a way as to be pivotal on the center axis of the operation section unit 3. In this case, an outer peripheral protuberance 69a of the rubber packing 69 is in close contact with the internal peripheral surface of the large diameter section 77 of the insertion. section joint section 73, and hence an appropriate resistance force exerts on the pivotal movement of the sheath unit 7.

To remove the sheath unit 7 from the operation section unit 3, for example, manually grasp and pull the sheath unit 7 opposite in direction to the insertion of the sheath unit 7 into the insertion section joint section 73. In this way, if a relatively large pulling force is exerted on the sheath unit 7, the snap fit arms 67, 67 are resiliently deformed and deflect inwardly. As a result of this, the protuberances 68 of the snap fit arms 67, 67 are pulled into the small diameter section 78 of the insertion section joint section 73. The protuberance 68 of each of the snap fit arms 67, 67 is disengaged from the rear end surface of the insertion joint section 73 of the fixed handle 70, whereby the sheath unit 7 is disconnected from the insertion section joint section 73. In this way, the sheath unit 7 is removed from the operation section unit 3.

At this time, the cone angle θ₂ of the second slop 68b on the root side of the protuberance 68 of each snap fit arm 67 is set to 45 degrees in this embodiment. The sheath unit 7 is pulled out of the operation section unit 3 by means of a force which is larger than the elastic force of the snap fit arms 67, 67 and a frictional force developing between the second slope 68b on the root side of the protuberance 68 of each snap fit arm 67 and the rear end surface of the insertion section joint section 73 of the fixed handle 70.

On the assumption that the cone angle θ₂ of the second slope 68b on the root side of the protuberance 68 of each of the snap fit arms 67 is set more than 90 degrees, it becomes necessary to disengage the protuberance 68 of each of the snap fit arms 67, 67 from the rear end surface of the insertion section joint section 73 of the fixed handle 70 by forcibly bending the snap fit arms 67, 67 inwardly using means of some kind, for example, pinching the leading ends of the snap fit arms 67, 67 by the fingers.

If the cone angle θ₂ of the second slope 68b on the root side of the protuberance 68 of each of the snap fit arms 67, 67 is reduced, if the elastic force of the snap fit arms 67, 67 is reduced, or if the diameter of the protuberance 68 is reduced, it will become possible to reduce the force necessary for pulling the sheath unit 7 out of the operation section unit 3. Conversely, if the cone angle θ₂ of the second slope 68b on the root side of the protuberance 68 of each of the snap fit arms 67, 67 is increased, if the elastic force of the snap fit arms 67, 67 is increased, or if the diameter of the protuberance 68 is increased, it will become possible to increase the force necessary for pulling the sheath unit 7 out of the operation section unit 3.

Further, if the outer diameter of the protuberance 68 of each of the snap fit arms 67, 67 is increased, if the cone angle θ₁ of the first slope 68a on the leading end side of the protuberance 68 of each snap fit arm 67 is increased, and if the elastic force of the snap fit arms 67, 67 is increased, the force needed when the sheath unit 7 is attached to the operation section unit 3 becomes increased. If the reverse of the above adjustments is made, the attachment force becomes smaller.

If the relationship between the cone angle θ₂ of the second slope 68b on the root side of the protuberance 68 of each snap fit arm 67 and the cone angle θ₁ of the first slop 68a on the leading end side of the protuberance 68 of each snap fit arm 67 is controlled, it is possible to control the relationship between the force necessary for attaching the sheath unit 7 to the operation section unit 3 and the force necessary for pulling the sheath unit 7 out of the operation section unit 3. Specifically, if the cone angle θ₁ of the first slope 68a on the leading end side of the protuberance 68 of each snap fit arm 67 is set larger than the cone angle θ₂ of the second slope 68b on the root side of the protuberance 68 of each snap fit arm 67, the attachment force becomes larger than the pull-out force. Conversely, if the cone angle θ₂ of the second slope 68b on the root side of the protuberance 68 of each snap fit arm 67 is set larger than the cone angle θ₁ on the leading end side of the protuberance 68 of each snap fit arm 67, the pull-out force becomes larger than the attachment force.

In this way, If a cone angle θ, which is a difference between the cone angle θ₁ of the first slope 68a on the leading end side of the protuberance 68 of the snap fit arm 67 and the cone angle θ₂ of the second slope 68b on the root side of the protuberance 68 of each snap fit arm 67, is optionally changed, the attachment and pull-out forces can be controlled. Although the number of the snap fit arms 67 is two in this embodiment, the number may be changed to three, four, or more.

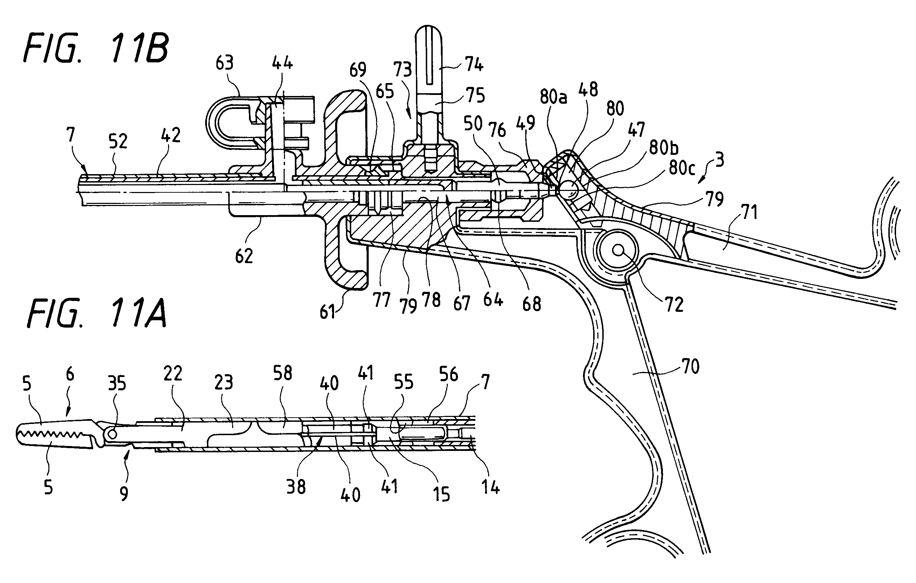

The method of attaching the treatment section drive unit 9 to a assembly unit, which is made by connecting the sheath unit 7 to the operation section unit 3 in the manner as previously mentioned, will be described. To begin with, the treatment section drive unit 9 is inserted into the inside of the sheath unit 7 through an opening on the leading end side of the sheath unit 7. At this time, the treatment section drive unit 9 is inserted into the inside of the sheath unit 7 from the connection ball 47 provided at the rear end portion of the sheath unit. When the treatment section drive unit 9 is inserted into the sheath unit 7, the movable operation handle 71 is previously held in a state in which the handle 71 is rotated counterclockwise until it comes into contact with the rubber cap 76 as shown in Fig. 11B.

The treatment section drive unit 9 inserted into the sheath unit 7 is pressed until the connection ball 47 is pushed out from the aperture 76a of the rubber cap 76 provided at the rear end portion of the insertion section joint section 73 of the operation section unit 3. If the projection 23 of the treatment section drive unit 9 butts against the projection 58 of the sheath unit 7, and if they do not mesh with each other during the insertion of the treatment section drive unit 9 into the sheath unit 7, the treatment section drive unit 9 and the sheath unit 7 are out of alignment with each other in the direction of rotation at a proper inserting position. In this case, before the treatment section drive unit 9 is inserted to the proper inserting position, the projection 23 of the treatment section drive unit 9 butts against the projection 58 of the sheath unit 7, which prevents the further insertion of the treatment section drive unit 9. In this way, it becomes impossible to correctly insert the treatment section drive unit 9 into the inserting position. In this case, the amount of projection of the connection ball 47 provided at the rear end portion of the treatment section drive unit 9 out from the rubber cap 76 of the operation section unit 3 becomes smaller. In this state, even if the movable operation handle 71 is rotated counterclockwise until it comes into contact with the rubber cap 76 as shown in Fig. 11B, it is impossible to completely hold the connection ball 47 within the ball guide hole 80b of the joint groove 80 of the movable operation handle 71.

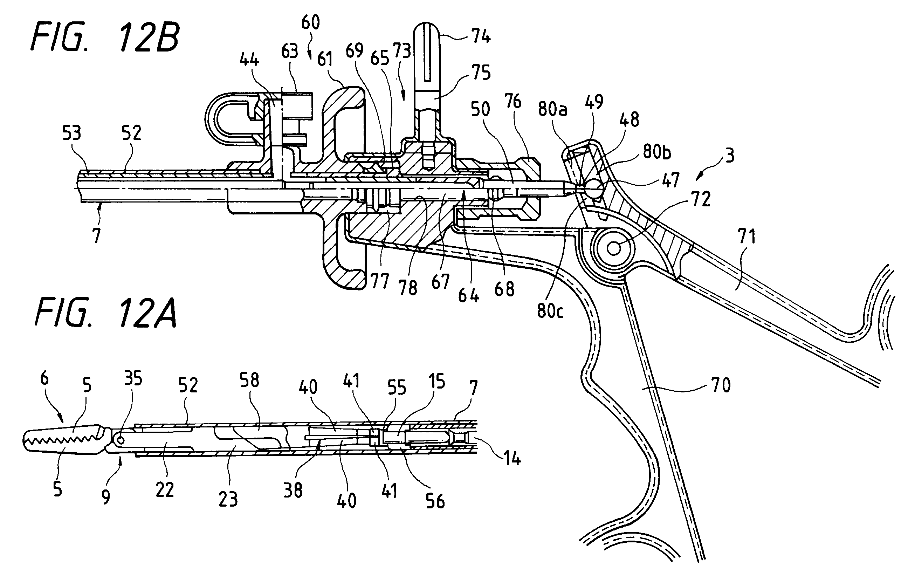

To prevent this, if the treatment section drive unit 9 is rotated around the axis with respect to the sheath unit 7, they are placed in alignment with each other in the direction of rotation at the proper fitting position, whereby the projection 23 of the treatment section drive unit 9 and the projection 58 of the sheath unit 7 appropriately mesh with each other, as shown in Fig. 12A.

After the treatment section drive unit 9 has been properly aligned in the direction of rotation, the actuation shaft 12 is retained at a push-out position. The treatment section drive unit 9 is further inserted into the inside of the sheath unit 7 while both of the grasping members 5 of the treatment section 6 are held in the closed position.

As shown in Fig. 12B, if the projection 23 of the treatment section drive unit 9 and the projection 58 of the sheath unit 7 slightly mesh with each other while they are appropriately aligned with each other in the direction of rotation at the proper fitting position, the amount of projection of the connection ball 47 provided at the rear end portion of the treatment section drive unit 9 out from the rubber cap 76 of the operation section unit 3 becomes larger compared with the amount of projection obtained in the case as shown in Fig. 11B.

Further, as shown in Fig. 12A, the moment at which the treatment section drive unit 9 is inserted into the sheath unit 7 until the projection 23 of the treatment section drive unit 9 starts to mesh with the projection 58 of the sheath unit 7, the protuberances 41 of the snap fit section 38 of the treatment section drive unit 9 come into contact with the slope 55a on the leading end side of the inner protuberance 55 of the connection pipe 53 of the sheath unit 7, whereby each of the snap fit arms 40 deflects inwardly.

If the treatment section drive unit 9 is pushed into the sheath unit 7 further more, the protuberances 41 completely went over the internal protuberance 55 and into the holding section 56. Thereafter, the protuberances 41 come into contact with the step 57, that is, the slope 55b on the handle side of the internal protuberance 55.

As a result of this, the leading end surface of the projection 58 of the sheath unit 7 butts against the rear end surface of the projection 23 of the treatment section drive unit 9, so that the projections 58 and 23 completely mesh with each other. In this way, the treatment section drive unit 9 does not reach the proper fitting position before being inserted until the projection section 23 of the treatment section drive unit 9 completely meshes with the projection 58 of the sheath unit 7.

Further, the connection ball 47 of the treatment section drive unit 9 is engaged with the joint groove 80 of the operation section unit 3 at the same time the treatment section drive unit 9 is inserted to the proper fitting position within the sheath unit 7. In other words, the leading end face of the projection 58 of the sheath unit 7 butts against the rear end face of the projection 23 of the treatment section drive unit 9, and the sheath unit 7 and the treatment section drive unit 9 are positionally adjusted so as to be aligned with each other in the direction of rotation until they completely mesh with each other. Thereafter, the connection ball 47 of the treatment section drive unit 9 is inserted into the entrance aperture 80a of the joint groove 80 of the operation unit 3 during the course of the insertion of the treatment section drive unit 9 to the proper fitting position within the sheath unit 7.

The connection ball 47 pushes the joint groove 80 of the operation section unit 3 in the direction of the handle as the treatment section drive unit 9 is further inserted toward the proper fitting position within the sheath unit 7. As a result of this, the movable operation handle 71 is rotated clockwise in Fig. 12B, and the connection ball 47 of the connection member 46 is completely inserted into the ball guide hole 80b of the joint groove 80 of the movable operation handle 71, as shown in Fig. 12B.

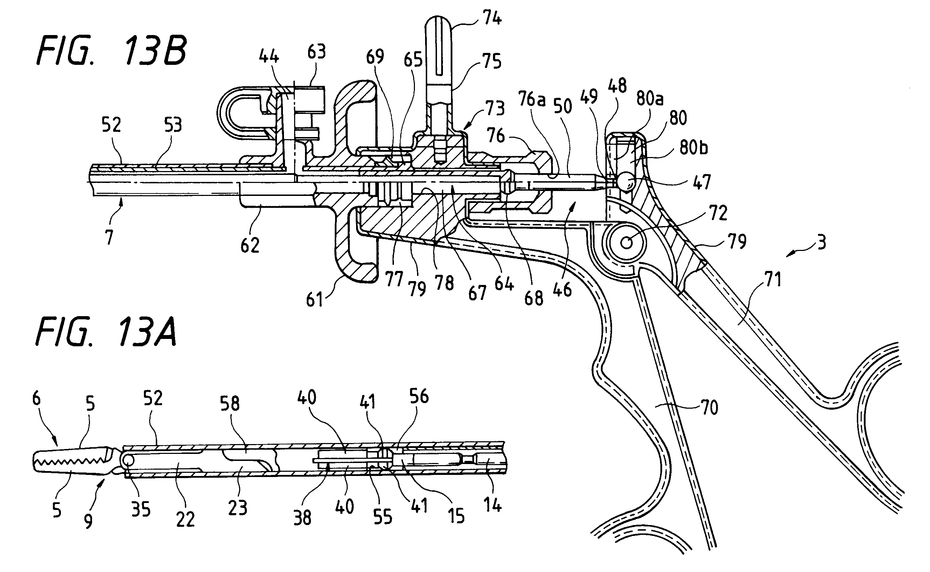

If the movable operation handle 71 is rotated clockwise further while being in the state as shown in Fig. 12B, the connection ball 47 of the connection member 46 is pulled in the direction of the handle by means of the ball guide hole 80b. As a result of this, the small diameter section 48 and the intermediate diameter section 49 of the connection member 46 enter, in that order, the connection member guide section 80, as shown in Fig. 13B, whereby the attachment of the assembly unit, made by connection of the sheath unit 7 to the operation section unit 3, to the treatment section drive unit 9 is finished. Thus, the assembly of the grasp type forceps 1 is completed.

As shown in Figs. 11A and 11B, it is set that the connection ball 47 of the connection member 46 does not completely enter the joint groove 80 or the ball guide hole 80b of the movable operation handle 71 when the projection 23 of the treatment section drive unit 9 butts against the projection 58 of the sheath unit 7. By virtue of this setting, if the connection ball 47 of the connection member 46 enters the ball guide hole 80b of the movable operation handle 71 while the projection 23 of the treatment section drive unit 9 and the projection 58 of the sheath unit 7 remain in butted contact with each other, and if the movable operation handle 71 is rotated clockwise while the projections 23 and 58 remain in contact with each other, the projection 23 of the treatment section drive unit 9 and the projection 58 of the sheath unit 7 are prevented from being forcefully brought into contact with each other, and hence they are not damaged.

While the treatment section drive unit 9 is inserted and attached to the proper fitting position within the sheath unit 7, the actuation shaft 12 of the treatment section drive unit 9 is retained while being inserted into the snap fit arms 67, 67 of the sheath unit 7. In this case, the outer diameter of the pipe cover 45 of the actuation shaft 12 inserted into the inside of the snap fit arms 67, 67 and the outer diameter of the heat-shrinkable tube 51 covering the connection member 46 are substantially the same as the inner diameter of the snap fit arms 67, 67 of the sheath unit 7. Therefore, in this state, the actuation shaft 12 hinders the movement of the snap fit arms 67, 67 in the inward direction. Accordingly, it is impossible for the snap fit arms 67, 67 to deflect inwardly, and hence it is possible to reliably prevent the removal of the sheath unit 7 from the operation section unit 3.

Specifically, if the operation section unit 3, the sheath unit 7, and the treatment section drive unit 9 are assembled in a proper assembly state, it is possible to prevent the disengagement of the sheath unit 7 from the operation section unit 3. This eliminates a fear of disengagement of the sheath unit 7 from the operation section unit 3 during the course of medical operation.

It is possible to change the force, which is needed to attach and detach the treatment section drive unit 9 to and from the sheath unit 7, in a variety of ways by optionally changing the cone angles of the slope 41a on the leading end side of the protuberance 41 of the snap fit arm 40, the slope 41b on the handle side of the same, the slope 55a on the leading end side of the inner protuberance 55 of the connection pipe 53 of the sheath unit 7, and the slope 55b on the handle side of the same with respect to the center axis, or by changing the number of snap fit arms 40 or the elasticity of the same. This is the same as the previously mentioned fact that the force necessary for the attachment and detachment of the sheath unit 7 to and from the operation section unit 3 is changeable in a variety of ways by changing various conditions such as the snap fit arms 67 and protuberances 68 of the snap fit section 64 of the sheath unit 7.

How the grasp type forceps 1 grasps the tissue H will now be described. When the grasping members 5, 5 of the treatment section 6 are closed, the moveable operation handle 71 is rotated clockwise. In this case, the connection ball 47, the handle-side operation rod 14, and the leading-end-side operation rod 13 of the treatment section drive unit 9 are pulled in the direction of the handle as the operation handle 71 is rotated. As a result of this, the forceps opening and closing member 16 is forcefully moved in the direction of the handle, and the slot side surface 20a on the leading end side of each of the pin slide slots 20 pushes each guide pin 36 in the direction of the handle. Resultantly, the guide pins 36, 36 are guided along the pin slide slots 20, 20, and the grasping members 5, 5 of the treatment section 6 are pivotally moved in a closing direction around the caulking pin 35 together with the movement of the guide pins 36.

At this time, since the guide pins 36 move in the direction of the handle when pushed toward the handle by the slot side surface 20a on the leading end side of the pin slide slot 20, the elongated holes 34 of the link arms 33 slide along the caulking pin 35, whereby the grasping members 5, 5 move in the direction of the handle. In this case, the cam mechanism, made up of the caulking pin 35 which serves as the center for the opening and closing action of the grasping members 5, 5 and the elongated holes 34, 34 of the grasping members 5, 5 into which the caulking pin 35 is inserted, controls the opening and closing action of the grasping members 5, 5 in the following manner as the grasping members 5, 5 are moved in the direction of the handle. Namely, if the shapes of the pin slide slots 20, 20 and the elongated holes 34, 34 of the grasp type forceps 1 of this embodiment are arbitrarily set, it is possible to, for example, make the grasp sections 31, 31 of the grasping members 5, 5 substantially parallel to each other immediately before the grasping members 5, 5 are completely closed during the course of the closing action of the forceps grasping members 5, 5, and to horizontally draw the tissue H to be grasped in the direction of the handle as the movable operation handle 71 is further rotated clockwise from the position where the grasping members 5, 5 are rendered parallel to each other.

When the grasping members 5, 5 are progressively closed as shown in Figs. 5A and 5B, the elongated holes 34, 34 slidably move along the caulking pin 35. At this time, the distance between the caulking pin 35 and the guide pins 36, 36 is also progressively extended, as shown in Figs. 15A and 15B, compared with the distance obtained when the grasping members 5, 5 of the treatment section 6 are closed. Associated with these variations in the distance, the distance between the caulking pin 35 and the leading ends of the grasps 31, 31 of the grasping members 5, 5 becomes shorter. On the assumption that the force exerted on the guide pins 36, 36 from the pins slide slots 20, 20 is constant, the grasping force acting on the leading ends of the grasp sections 31, 31 eventually increases progressively by virtue of leverage. For this reason, compared with a commonly used conventional configuration in which the target tissue H is grasped by pivotally rotating the grasping members 5, 5 of the treatment section 6 around the caulking pin 35, the grasping force obtained in this embodiment is increased. Not only the grasping force at the leading ends of the grasp sections 31, 31 of the grasping members 5, 5 of the treatment section 6 increases, but also the substantial grasping capability of the grasping members 5, 5 increases, because the grasp sections 31, 31 grasp the tissue H substantially horizontally so as to draw the tissue in the direction of the handle as previously mentioned when the grasp type forceps 1 grasp the target tissue H.

When the grasp members 5, 5 of the treatment section 6 are closed while grasping the tissue H, the grasping force exerts on the leading ends of the grasp sections 31, 31 of the grasping members 5, 5. However, the clearance S₁ is formed in the pin slide slots 20 of the grasp member connecting section 18 in the direction of the leading end in relation to the position of the guide pins 36 the moment at which the grasping members 5, 5 are closed. By virtue of this clearance S₁, the guide pins 36, 36 are not brought into contact with the end portions on the leading end side of the pin slide slots 20, 20. For this reason, after the leading ends of the grasping members 5, 5 have come into contact with each other, it is possible to draw the actuation shaft 12 in the direction of the handle to a further extent in correspondence to the distance equal to the clearance S₁. Accordingly, it is possible to sufficiently transmit the force exerted on the movable operation handle 71 to the leading ends of the grasping members 5, 5, and also the grasping members 5, 5 can forcefully and reliably grasp the tissue H similar to a thin film.

The width of this clearance S₁ may be arbitrarily controlled depending on the magnitude of the force to be exerted on the grasping members 5, 5. In some cases, the pin slide slots 20 may be opened through to the leading end side of the forceps opening and closing member 16.

Moreover, the grasping members 5, 5 are manufactured by forging metallic materials such as stainless materials, and hence a flow, that is, so-called fiber flow appears in a specific direction in the grasping members as one of effects resulting from improvements on the material by means of forging, as previously described in the explanation of the operation of the forceps. By virtue of this effect, not only the mechanical strength of the material but also the elasticity of the same are particularly improved in the direction of the fiber flow. In this embodiment, the material is forged so as to bring about the fiber flow in the longitudinal direction of the grasping members 5, 5. The gap 31a is formed for ensuring an opening in the contact area between the grasp sections 31, 31 of the grasping members 5, 5 when the grasping members 5, 5 are closed. By virtue of this configuration, if the movable operation handle 71 is rotated clockwise further from the position where the grasping members 5, 5 are closed, the grasp sections 31, 31 of the grasping members 5, 5 made of forged metallic material or intermediate areas between the grasp sections 31, 31 and the link arms 33, 33 are deflected, whereby the gap 31a is closed. As a result of this, it is possible to grasp the tissue H more reliably.

Moreover, the grasp sections 31, 31 of the grasping members 5, 5 or the intermediate areas between the grasp sections 31, 31 and the link arms 33, 33 are deflected when the tissue H is grasped, as previously mentioned. Therefore, it is possible to grasp the tissue H more reliably by resiliently deforming the grasping members 5, 5 in accordance with the shape of the grasped tissue H. Assume now that the mechanical strength and elasticity of the grasping members 5, 5 are optionally set by changing the thickness of the grasp sections 31, 31 or changing molding conditions such as a forging ratio when the grasping members 5, 5 are forged, in the manner as previously mentioned, in agreement with the mechanical strength of the tissue H grasped by the grasping members 5, 5. On this assumption, if an excessive force is exerted on the movable operation handle 71 when the tissue H is grasped, the tissue H might not be damaged, and therefore the tissue H can be grasped intact.

In this embodiment, the entrance aperture 80a and the ball guide hole 80b of the movable operation handle 71 are designed so as to become substantially perpendicular to the axis of the connection member 46, as shown in Fig. 13A, when the grasping members 5, 5 are closed as shown in Fig. 13B. With this configuration, the direction of the force, which is exerted on the connection member 46 via the connection ball 47 within the ball guide hole 80b from the movable operation handle 71 when the movable operation handle 71 is rotated clockwise in Fig. 1, can be put in alignment with the direction of the force for pulling the connection member 46 toward the handle in the direction of the axis. In this way, it is possible to obtain the force for closing the grasping members 5, 5 most effectively. Therefore, even if the grasping members 5, 5 are substantially closed as if to grasp the thin-film-like tissue H, it is possible for the grasping members 5, 5 to reliably grasp the thin-film-like tissue H.

In this embodiment, the ball guide hole 80b and the connection member guide section 80c are set so as to become perpendicular when the grasping members 5, 5 are nearly closed. However, the ball guide hole 80b and the connection member guide section 80c may be set so as to become perpendicular when the grasping members 5, 5 are opened to an arbitrary angle depending on the type of the tissue H to be grasped.

Further, the small diameter section 48 of the connection member 46 is set so as to be smaller in breaking force than the other parts. The small diameter section 48 of the connection member 46 is designed so as to first break even if the force larger than required (i.e., the force for rotating the operation handle clockwise in Fig. 1) is exerted on the movable operation handle 71 when the grasping members 5, 5 grasp the tissue H or the like. By virtue of this configuration, it is possible to prevent the force larger than required from acting on other parts. Moreover, in the case of this configuration, if the connection member 46 is manufactured by machining, the small diameter section 48 might buckle when exfoliating the tissue H as will be described later. However, if forceps are manufactured by forging metallic materials in the same manner as the grasp type forceps 1 of this embodiment, the elasticity of the forceps is improved as has been already described, and hence the small diameter section 48 is prevented from buckling.

Furthermore, the minimum cross-sectional area section 83 is provided in the vicinity of the root of the guide pin 36 on the leading end side of the link arm 33 of each grasping member 5, as shown in Fig 6A. In case an excessive force acts on the guide pin 36 from the side surface of the pin slide slot 20 of the forceps opening and closing member 16 when the grasping members 5, 5 are opened and closed, the minimum cross-sectional area section 83 can first break before any other parts of the grasping member 5 break. For this reason, there is no fear of the fracture of parts other than the minimum cross-sectional area section 83 of the link arm 33 of the grasping member 5.

The forceps opening and closing member 16, the link arm 33 of the grasping member 5, and the leading end cover 21 are covered with the insulating tube 52, and therefore there is no fear of dropping of broken pieces of the guide pin 36, or the like, out of the insulating tube 52 in case the minimum cross-sectional area section 83 of the link arm 33 breaks while the forceps 1 are in use.

When the grasping members 5, 5 are closed, and when the guide pin 36 of the link arm 33 is brought into contact with the slot side surface 20a of the pin slide slot 20, as shown in Fig. 6B, the backlash S₂ is formed, as required, between the guide pin 36 and the slot side surface 20b on the handle side of the pin slide slot 20. By virtue of this configuration, as shown in Fig. 6B, when the movable operation handle 71 is rotated counterclockwise so as to push the actuation shaft 12 and the forceps opening and closing member 16 toward the leading end of the forceps in order to open the grasping members 5, 5 while the guide pin 36 of the link arm 33 is brought into contact with the slot side surface 20a on the leading end side of the pin slide slot 20, the forceps opening and closing member 16 is previously shifted toward the leading end of the forceps in correspondence to the interval of the backlash S₂, and thereafter the guide pin 36 can be brought into contact with the slot side surface 20b on the handle side of the pin slide slot 20.

For this reason, the leading end small diameter section 15c of the operation rod connection member 15 can be inserted into the inside of the snap fit arms 40 in correspondence to the backlash S₂ before the guide pin 36 is pushed by the forceps opening and closing member 16, as shown in Fig. 14B. Accordingly, the leading end small diameter section 15c of the operation rod connection member 15, which is inserted into the inside of the snap fit arms 49 while the grasping members 5, 5 are still closed, can prevent the snap fit arms 40 from deflecting inwardly. As a result of this, the protuberances 41 of the snap fit arms 40 also become impossible to shift inwardly. Therefore, it is possible to keep the slope 41a on the leading end side of the protuberance 41 remaining in contact with the slope 55b one the handle side of the inner protuberance 55 of the sheath unit 7, and hence it is possible to prevent the treatment section drive unit 9 from disengaging from the sheath unit 7.

The prevention of the disengagement of the treatment section drive unit 9 from the sheath unit 7 has the following advantageous effects. For example, as shown in Fig. 14A, where a sample tissue H is exfoliated from the tissue H by opening the grasping members 5, 5 after the forceps 1 have been inserted into a gap H₁ in the tissue H while the grasping members 5, 5 are closed, a force for closing the grasping members 5, 5 are exerted on the grasping members 5, 5 from the living body tissue H. On the assumption that the backlash S₂ is not formed in the pin slide slot 20, and that the grasping members 5, 5 are opened by rotating the movable operation handle 71 counterclockwise in Fig. 1 after the forceps 1 have been inserted into the gap H₁ of the tissue H while the grasping members 5, 5 are closed, the actuation shaft 12 is pushed toward the leading end of the forceps, in other words, the treatment section drive unit 9 is pushed in the direction in which it leaves outside the sheath unit 7, and hence the snap fit arms deflect inwardly. As a result of this, the protuberance 41 goes over and disengages from the inner protuberance 55, and the treatment section drive unit 9 might disengage from the sheath unit 7. Contrary to this, if the backlash S₂ is formed in the pin slide slot 20 in the same manner as in this embodiment, the leading end small diameter section 15c of the operation rod connection member 15 can be inserted into the inside of the snap fit arms 40 while the grasping members 5, 5 are closed. Hence, the treatment section drive unit 9 will not disengage from the sheath unit 7 while the tissue H is exfoliated, which ensures safe operation.

The leading end of the operation rod 13 is screwed into the forceps opening and closing member 16, whereas the rear end of the operation rod 13 is also screwed into the operation rod connection member 15. If the lengths of the screwed portions of the operation rod 13 are respectively controlled, the position of the operation rod connection member 15 with respect to the snap fit arms 40, i.e., the position of the leading end small diameter section 15c of the operation rod connection member 15 can be controlled in the direction of the axis of the sheath unit 7.

Further, the forceps opening and closing member 16 and the leading-end-side operation rod 13 may be integrated together, or the operation rod connection member 15 and the handle-side operation rod 14 may be integrated together, or the leading-end-side operation rod 13, the handle-side operation rod 14, and the operation rod connection member 15 may be integrated together. In any one of these cases, they are integrated by forging stainless steel.

The contact surface 36a on the leading end side of the guide pin 36 of each grasping member 5 is formed so as to be substantially the same in a radius of curvature as the slot side surface 20a on the leading end side of the pin slide slot 20, as shown in Fig. 6B. On the other hand, the contact surface 36b on the handle side of the guide pin 36 is formed so as to be substantially the same in a radius of curvature as the slot side surface 20b on the handle side of the pin slide slot 20. Compared with the case of a forceps in which a guide pin having a circular cross section is used, this embodiment makes it possible to increase a contact area between the contact surface 36a of the guide pin 36 of the grasping member 5 and the slot side surface 20a of the pin slide slot 20, and a contact area between the contact surface 36b of the guide pin 36 and the slot side surface 20b of the pin slide slot 20. Therefore, the abrasion of the contact surfaces 36a and 36b of the guide pin 36 of each grasping member 5, which are brought into slidable contact with the pin slide slot 20 when the grasping members 5, 5 are opened and closed, is reduced.

The grasping members 5, 5 and the forceps opening member 16 are manufactured by forging metallic materials such as stainless steel, and therefore the mechanical strength and surface hardness of the grasping member and the forceps opening member are improved by virtue of the previously mentioned improvements on the material. Compared with forceps manufactured by ordinary machining, the forceps of this embodiment are less apt to be deformed and damaged, thereby providing high durability and reliable operation.

The contact surfaces 36a and 36b of the guide pin 36 of each grasping member 5 may be linear, and radii of the curvature of the contact surfaces are arbitrarily selected as required.

When the grasping members 5, 5 of the treatment section 6 are opened, the movable operation handle 71 is rotated counterclockwise further from the state of the movable operation handle 71 shown in Fig. 1. In this case, the actuation shaft 12 of the treatment section drive unit 9 is pushed toward the leading end of the forceps together with the rotation of the movable operation handle 71.

Associated with the movement of the actuation shaft 12, the guide pins 36 of the grasping members 5 move to the rear end portion of the pin slide slot 20 along the pin slide slot 20, whereby the grasping members 5, 5 become progressively opened. At this time, the guide pin 36 is pushed forward by the slot side surface 20b on the handle side of the pin slide slot 20 at the leading end portion of the forceps opening and closing member 16 as the actuation shaft 12 is moved. As a result of this, the elongated holes 34 of the grasping members 5 move toward the leading end side of the forceps along the caulking pin 35 as the grasping members 5, 5 are pushed toward the leading end side of the forceps via the guide pins 36. The grasping members 5, 5 are opened much further, and they are fully opened at last as shown in Fig. 15A.

Compared with conventional forceps in which the grasping members 5,5 are simply opened around the caulking pin 35, the movement of the elongated holes 34, 34 toward the leading end of the forceps allows the base portions of the grasp sections 31, 31 of the grasping members 5, 5 to open much wider in this embodiment. Therefore, much larger exfoliating action of the grasping members is made available during the exfoliation of the tissue H which will be described later. It is possible to reliably grasp large tissue H or slippery tissue H.

The leading end small diameter section 15c of the operation rod connection section 15 in the treatment section drive unit 9 completely enters the inside of the snap fit arms 40 while the grasping members 5, 5 are fully opened, as shown in Fig. 15B. The snap fit arms 40 do not deflect inwardly, and therefore the protuberances 41 do not disengage from the inner protuberance 55 within the sheath unit 7. For this reason, the treatment section drive unit 9 does not disengage from the sheath unit 7 by means of a force developed when the grasping members 5, 5 are opened by rotating the movable operation handle 71 counterclockwise during the course of the operation for opening the grasping members 5, 5.

Since the backlashes S₂, S₂ are respectively ensured between the slot side surfaces 20b on the handle side of the pin slide slots 20 and the guide pins 36, the leading end small diameter section 15c of the operation rod connection member 15 remains inserted within the inside of the four snap fit arms 40 even when the grasping members 5,5 are closed. Therefore, the treatment section 9 does not disengage from the sheath unit 7 during the closing of the grasping member 5, 5.

To grasp the tissue H, the grasping members 5, 5 are opened in agreement with the size of the tissue H, and the movable operation handle 71 is rotated clockwise. As a result of this, the grasping members 5, 5 are closed to grasp the tissue H.

A connector of a power transmission code from a high-frequency power supply (not shown) is attached to the electrode pin 74 of the operation section unit 3, and the high-frequency current is fed to the electrode pin when necessary. As a result of this, power is applied to the grasping members 5, 5 by way of the insertion section joint section 73, the snap fit section 64, the inner pipe 42, the connection pipe 53, the snap fit section 38, the leading end cover 21, and the caulking pin 35, in that order. With use of this power, the tissue H grasped between the grasping members 5, 5 is burned, or bleeding from the tissue is stanched.

The link arm 33 of the grasping member 3, the forceps opening and closing member 16, and the leading end cover 21 are completely covered with the insulating tube 52, and therefore there is no possibility of leak of the high-frequency current from these areas when the high-frequency current is applied. The high-frequency current flows only to the grasping members 5, 5, and hence it is possible to reliably burn the tissue H with which the grasping members 5, 5 are in contact. In this way, it is possible to prevent accidental unintended burning of tissue around the target lesion occurring when the leading cover 21, the forceps opening and closing member 16, or the link arm 33 of the grasping member 5 unexpectedly comes into contact with tissue surrounding the lesion during the treatment.

While the tissue H is being forcefully grasped between the grasping members 5, 5, a strong force for bringing about clockwise rotation acts on the moveable operation handle 71. By means of this force, the connection member 46 is forcefully pulled toward the handle side of the forceps, and the treatment section drive unit 9 is also pulled toward the handle side. As a result of this, the connection pipe 53 of the sheath unit 7 remaining in contact with the leading end cover 21 of the treatment section drive unit 9 is forcefully pushed to the handle side of the forceps, and therefore the sheath unit 7 is pressed against the insertion section joint section 73 of the fixed handle 70. Eventually, the butting surface 65a in the rear end portion of the connection pipe 65 of the snap fit section 64 of the sheath unit 7 is strongly brought into contact with the butting surface 77a at the step between the large diameter section 77 and the small diameter section 78 in the insertion section joint section 73 of the fixed handle 70.

At this time, a strong frictional force develops, as a result of the contact, between the butting surface 65a in the rear end portion of the connection pipe 65 and the butting surface 77a of the insertion section joint section 73. By means of this frictional force, it is possible to retain the sheath unit 7 in such a way that the sheath unit 7 cannot easily rotate around the axis of the operation section unit 3.

The sheath unit 7 cannot easily rotate around the insertion axis of the forceps 1 during the grasping operation using the grasping members 5, 5 by means of the frictional force developed between the butting surface 65a in the rear end portion of the connection pipe 65 and the butting surface 77a of the insertion section joint section 73. However, in cases other than the grasping operation, it is possible to rotate the sheath unit 7 by rotating the rotary control 61 by hands or fingers. At this time, the projection 58 of the leading end cover 21 of the sheath unit 7 is meshing with the projection 23 of the leading end cover 21 of the treatment section drive unit 9, and therefore the sheath unit 7 rotates along with the treatment section drive unit 9 when the sheath unit 7 rotates with respect to the operation section unit 3.

To remove the treatment section drive unit 9 from the sheath unit 7 for cleansing or sterilization, the movable operation handle 71 is slightly rotated clockwise while the grasping members 5, 5 are completely closed as shown in Figs. 5A and 5B. Associated with this removal operation, the actuation shaft 12 of the treatment section drive unit 9 is pulled to the handle side of the forceps, so that the leading end small diameter section 15c of the operation rod connection section 15 is completely removed to the outside from the snap fit arms 40. In this state, the sheath unit 7 is withdrawn from the treatment section drive unit 9 by pinching the grasping members 5, 5 by fingers or hands.

The slope 41a on the leading end side of the protuberance 41 of the snap fit arm 40 is pressed inwardly by means of the slope 55b on the handle side of the inner protuberance 55, whereby the snap fit arm 40 deflects inwardly, and the protuberance 41 goes over and disengages from the inner protuberance 55 in the direction of the leading end of the forceps. In this state, the treatment section drive unit 9 is pulled out further, and, at the same time, the movable operation handle 71 is rotated counterclockwise. While the movable operation handle 71 is in the state as shown in Fig. 11B, the connection ball 47 of the connection member 46 is removed from the ball guide hole 80b of the joint groove 80. As a result of this, the treatment section drive unit 9 can be completely pulled out of the sheath unit 7.

The tapered section 15d is formed on the end face on the leading end side of the leading end small diameter section 15c of the operation rod connection member 15, whilst the tapered section 40a, corresponding to the tapered section 15d of the operation rod connection member 15, is formed on the inner end face on the handle side of the protuberance 41 of the snap fit arm 40. With this configuration, when the treatment section drive unit 9 is pulled out of the sheath unit 7, the protuberance 41 is pushed inwardly by means of the slope 55b on the handle side of the inner protuberance 55, whereby the tapered section 40a of the snap fit arm 40 is brought into contact with the tapered section 15d of the operation rod connection member 15. Then, the tapered section 15d is pushed in the direction of the handle side of the forceps, which in turn makes it easy to push the leading end small diameter section 15c of the operation rod connection member 15 from the inside of the snap fit arms 40 toward the handle of the forceps. Eventually, the protuberances 41 become easy to deflect inwardly, and the sheath unit 7 can be easily pulled out of the treatment section drive unit 9.

Further, as shown in Fig. 14A, a protuberance 81 which slightly bulges to the outside is formed between the grasp section 31 of each grasping member 5 and the link arm 33 slightly apart from the edge of the insulating tube 52. This protuberance 81 is formed so as to be smaller than the outer diameter of the insulating tube 52 when the grasping members 5, 5 are closed.

The protuberance 81 prevents the formation of a clearance between the grasp section 31 and the link arm 33. When the forceps 1 are inserted into, or removed from, a tracheal mantle tube or an insertion aperture of a surgical instrument of an endocope (neither of them being shown in the drawings) while the grasping members 5, 5 are closed after the treatment section drive unit 9, the operation section 3, and the sheath unit 7 have been assembled, the presence of the protuberance 81 prevents an airtight sealing member, which is provided within the tracheal mantle tube and the insertion aperture of the surgical instrument of the endoscope, from entering the clearance between the grasp section and the link arm so as to hinder the insertion and removal action of the forceps.

It is commonly well known that a user has difficult of classifying a surgical instrument for operation purposes like forceps 1 of this invention at first sight. If the kinds of surgical instrument for operation use are increased, there is a possibility that a worker in charge of performing maintenance, such as cleansing and sterilization of the surgical instrument, cannot properly assemble the forceps 1 from disintegrated three units, namely, the sheath unit 7, the treatment section drive unit 9, and the operation section unit 3 in the case of the forceps 1 as disclosed herein.

To avoid such a possibility, the color of the rubber stopper 63 attached to the sheath unit 7 of the grasp type forceps 1, the rubber cap 76 attached to the operation section unit 3, and the pipe cover 45 attached to the treatment section drive unit 9 is arbitrarily set in agreement with the classification of a facility where the forceps 1 as disclosed herein are used, which allows color classification of surgical instruments. As a result of this, the user can identify the type of surgical instruments at a glance, and a worker in charge of performing maintenance of the grasp type forceps 1 can reliably assemble the forceps by combining separated units in a proper manner after the forceps have been disassembled.

So long as the sheath unit 7, the treatment section drive unit 9, and the operation section unit 3 of the forceps 1 can be identified from each other by means of color, any portion of each unit of the sheath unit 7, the treatment section drive unit 9, the operation unit 3 may be colored.

The forceps having the configuration as has been previously mentioned yield the following advantageous effects. Specifically, parts of the forceps 1, that is, a pair of grasping members 5, 5, the leading end operation rod 13 of the treatment section drive unit 9, the handle-side operation rod 14, the leading end cover 21, and the connection ball 47 are manufactured by forging metallic materials such as stainless steel, titanium, or aluminum. The properties of the material itself are improved by forging, and hence the mechanical strength of the parts made by forging can be also improved. Compared with parts manufactured by machining, the specific mechanical strength of parts made by forging can be improved, and the parts can be made in reduced size and weight. As a result of this, it is possible to improve a degree of freedom of design and durability of forceps. The elimination of machining from the steps of manufacturing the forceps 1 and the mass-production of the forceps using mold-forging result in improved productivity and reduced manufacturing cost as well as reduced effort and expense related to manufacturing.

The thickness of the leading cover 21 of the treatment section drive unit 9 can be reduced by virtue of the improved mechanical strength of the material, whereby it is possible to ensure a relatively wide internal space in the treatment section drive unit 9. This wide internal space makes it easy to cleanse the inside of the treatment section drive unit 9 when the forceps 1 are cleansed and sterilized, thereby resulting in improved cleansing and sterilizing characteristics of the forceps 1. Further, in addition to the improved cleansing and sterilizing properties of the forceps, it is possible to improve the durability of the forceps such as the prevention of abrasion of a cum mechanism by virtue of improved surface hardness of the material and the prevention of fracture of forged members by virtue of improved elasticity of the material.

The connection ball 47 of the treatment section drive unit 9 is manufactured of forged parts made of, for example, stainless steel, and hence it is possible to improve not only the mechanical strength of the connection ball 47 but also the elasticity of the connection member 46 by forging. For these reasons, compared with a machined connection ball, the connection ball 47 is less likely to buckle at an engaged area between the connection ball 47 and the ball guide hole 80b of the joint slot 80 of the movable operation handle 71 upon receipt of pressing forces in a downward direction and the direction of the leading end of the forceps from the peripheral area of the joint slot 80 of the movable operation handle 71 when the tissue H is exfoliated using the forceps 1.

The leading-end-side operation rod 13 and the handle-side operation rod 14 are manufactured by forging metallic materials such as stainless steel. When compared with an operation rod made by machining, the leading-end-side operation rod 13 and the handle-side operation rod 14 are less likely to break even if they received an excessive force, because the mechanical strength and elasticity of the material itself ca be improved. Moreover, the improved elasticity of material permits the realization of grasping of tissue in an intact manner and ease of practical use at the same time.

The forceps of this embodiment are separable into three units; namely, the sheath unit 7, the treatment section drive unit 9, and the operation section unit 3. Compared with conventional surgical instruments for operation purposes, the surgical instrument of this embodiment is superior in ease of cleansing and sterilization.

Figs. 16A to 16G show scissors type forceps according to a second embodiment of the present invention. This embodiment is different from the first embodiment in that the configuration of the treatment section 6 is modified. Specifically, instead of the grasping members 5, 5 of the first embodiment, a cutting treatment member 103 having a fixed blade 101 and a movable blade 102, as shown in Figs. 16A and 16B, is used for the treatment section 6.

One of the support arms 22 of the leading end cover 21 in the first embodiment is further extended in the direction of the leading end of the forceps, and the fixed blade 101 is connected to this extended end. The fixed blade 101 has a bow-shaped excising blade 104 formed on the upper surface thereof.

As shown in Fig. 16D, a caulking pin 105 is caulked between the fixed blade 101 and the leading end portion of the other support arm 22, and the movable blade 102 is pivotally supported by this caulking pin 105. The fixed blade 101 and the movable blade 102 are made by forging stainless steel, or the like.

The leading-end-side operation rod 13 of the actuation shaft 12 of the first embodiment is further extended toward the leading end of the forceps, and the base portion of the movable blade 102 is pivotally connected to this extended end of the operation rod 13 by caulking with use of a caulking pin 106, as shown in Fig. 16E. An elliptical section 107 is formed at the leading end of the operation rod 13. An elongated caulking hole 108 is formed in this elliptical section 107, and the caulking pin 106 is inserted into this caulking hole 108. A backlash S₂ is formed, as required, between the caulking pin 106 and the internal surface on the handle side of the caulking hole 108 in the elliptical section 107 of the operation rod 13.

As shown in Fig. 16F, a plane notch 109 is made in a lower part of the operation rod 13 to prevent the operation rod 13 from coming into contact with the inner peripheral surface of the leading end cover 21 when the movable blade 102 pivotally moves around the caulking pin 105. In other words, the movable blade 102 pivotally moves around the caulking pin 105 as a result of the movement of the operation rod 13 in the direction of its axis by opening and closing the movable operation handle 71 of the operation section-unit 3. At this time, although the operation rod 13 vertically moves within the leading end cover 21 associated with the pivotal movement of the movable blade 102, the presence of the flat notch 109 prevents the lower surface of the operation rod 13 from coming into contact with the inner peripheral surface of the leading end cover 21. The operation rod 13 is made by forging metallic materials such as stainless steel, and forging is particularly effective in making the flat notch 109 of the operation rod 13.

This embodiment is different from the first embodiment in that the leading end cover 21 and the snap fit section 38 are formed into one unit by forging metallic materials such as stainless steel. Further, the centering section 15b of the rod operation connection member 15 has two notches 43 for guiding the flow of water, as shown in Fig. 16G. The number of notches 43 and the shape of the same can be arbitrarily determined so long as the flow of water is ensured by the notches (the notch may have a curved shape instead of a plane shape). The forceps of this embodiment are the same in configuration as the forceps of the first embodiment, except for the above mentioned part. The same reference numerals are provided to designate the corresponding features in the first embodiment, and hence the explanation thereof will be omitted here for brevity.

The operation of the configuration of the forceps of this embodiment will be described. To begin with, when the surgical instrument for operation purposes of this embodiment is in use, the actuation shaft 12 travels back and forth in response to the opening and closing action of the movable operation handle 71 (that is, the rotation of the movable operation handle 71 in either a clockwise or counterclockwise direction), and the movable blade 102 opens and closes in relation to the fixed blade 101 as a result of the pivotal movement of the movable blade 102 around the caulking pin 105 associated with the movement of the leading-end-side operation rod 13 of the actuation shaft 12. The tissue H is cut off or unillustrated stitches are cut by means of the opening and closing action of the movable blade 102.

To open the closed movable blade 102, the movable operation handle 71 is rotated counterclockwise. Associated with the movement of the operation rod 13 toward the leading end of the forceps, the caulking hole 108 slides along the caulking pin 106 over the distance in correspondence to the backlash S₂ in the direction of the leading end of the forceps, whereby the caulking pin 106 is brought into contact with the internal surface on the handle side of the caulking hole 108. As with the first embodiment, even in this embodiment, the leading end of the leading end small diameter section 15c of the operation rod connection member 15 enters the inside of the snap fit arms 40 of the snap fit section 38, which hinders the deflection of the protuberances 41 in an internal direction. As a result of this, the treatment section drive unit 9 does not come off the insertion section unit while the movable blade 102 is opening from its closed state.

The movable blade 102 is pressed by the leading-end-side operation rod 13 by further pushing the actuation shaft 12 after the caulking pin 106 has come into contact with the inner peripheral surface on the handle side of the caulking hole 108, as a result of which the movable blade 102 starts to open around the caulking pin 105.

In the case of the forceps having the previously mentioned construction, the fixed blade 101 and the movable blade 102 are formed by forging metallic materials such as stainless steel, and therefore each of the blades 101 and 102 has a large surface hardness and is less susceptible to abrasion. Moreover, the previously mentioned fiber flow appears in the longitudinal direction of the treatment member 103 for cutting purposes, and hence the fixed and movable blades 101 and 102 possess elasticity with respect to the direction of rubbing action of the blades 101 and 102. If the movable blade 102 is made to rub against the fixed blade 101 to ensure the sharpness of the scissors type forceps, the fixed blade 101 and the movable blade 102 elastically bend to each other, whereby the rubbing force can be absorbed by the elastic bending action of the blades. Therefore, the excessive rubbing force is not exerted on the caulking pin 106, and the rubbed areas of the blades do not spread. For these reasons, it is possible to ensure the sharpness of the scissors type forceps over a long period of time.

Fig. 17 shows scissors type forceps according to a third embodiment of the present invention. This embodiment is different from the first embodiment in that a scissors-like treatment section 142 having a pair of scissors blades 141 is attached to the leading end of the treatment section drive unit 9 instead of the grasping members 5, 5 of the first embodiment. Areas corresponding to the elongated holes 34, 34 of the first embodiment are formed into a simple circular hole. The scissors blades 141 are connected to the caulking pin 35 inserted into the circular holes by caulking in such a way as to be pivotally movable around the caulking pin 35.

The leading edge of each of the scissors blades 141 is finished into a curved shape corresponding to the radius R from the center of the caulking pin 35 to the leading edge of each of the scissors blades 141. With this configuration, it is possible to prevent the edges of the scissors blades 141 from injuring the tissue H even if the scissors blades 141 further move pivotally in the closing direction from the closing position shown in Fig. 17. The scissors type forceps of this embodiment are the same in configuration as the forceps of the first embodiment, except for the above mentioned part. The same reference numerals are provided to designate the corresponding features of the first embodiment, and hence the explanation thereof will be omitted here for brevity.

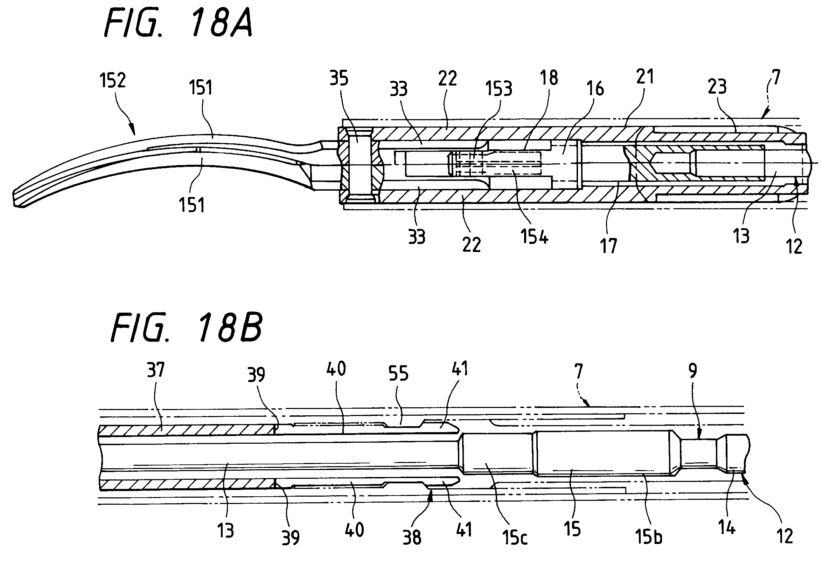

Figs. 18A and 18B show scissors-like forceps according to a fourth embodiment of the present invention. This embodiment is different from the third embodiment in that a treatment section 152 having a pair of bow-shaped scissors blades 151, 151 which are laid one over the other is attached to the leading end portion of the treatment section drive unit 9.

As shown in Fig. 18A, the grasp member connecting section 18 having a substantially rectangular and flat shape is attached to the leading end of the forceps opening and closing member 16, and a part of the grasp member connecting section 18 is sandwiched between the link arms 33 of the scissors blades 151. A narrow-width section 153 having a width slightly narrower than the interval between the link arms 33 is formed in the part of the grasp member connecting section 18 that is sandwiched between the link arms 33. A wider-width section 154 having a width substantially the same as the interval between the link arms 33 is formed on the other side of the grasp member connecting section 18 with respect to the narrow-width section 153, that is, the wider-width section 154 is formed in the area on the handle side of the grasp member connection section 18. This embodiment is the same in configuration as the third embodiment, except for the above mentioned point. The same reference numerals are provided to designate the corresponding features of the first embodiment, and hence the explanation thereof will be omitted here for brevity.

If the scissors blades 151, 151 of the forceps having the above-mentioned configuration are opened, a small frictional force develops between the narrow-width 153 and the link arms 33 when the actuation shaft 12 of the treatment section drive unit 9 advances in the direction of the leading end of the forceps while the narrow-width section 153 of the grasp member connecting section 18 of the forceps opening and closing member 16 is being sandwiched between the link arms 33. By virtue of this small frictional force, the actuation shaft 12 can smoothly advance in the direction of the leading end of the forceps (that is, the scissors blades 151, 151 can open smoothly). The moment at which the handle-side ends of the link arms 33 reach to the step between the narrow-width section 153 and the wider-width section 154 of the grasp member connecting section 18 of the forceps opening and closing member 16, the end and areas in the vicinity of the end of the leading end small diameter section 15c of the operation rod connection member 15 enters the inside of the protuberances 41.

In other words, the opening action of the scissors blades 151 is kept smooth until the leading end portion of the leading end small diameter section 15c enters the inside of the protuberances 41. When the scissors blades 151, 151 are opened, the treatment section drive unit 9 is pushed toward the leading end of the forceps as a result of the opening action of the blades, because a frictional force developed between the forceps opening and closing member 6 and the link arms 33 when the scissors blades 151, 151 are opened is excessively strong. In this way, the treatment section drive unit 9 is prevented from slipping off the sheath unit 7, and hence the opening action of the scissors blades 151, 151 can be reliably effected.

Further, in this embodiment, a manufacturer's name, a model number, or the like, may be inscribed on the side surface of the scissors blades 151, 151 of this embodiment using electrolytic marking or laser marking to make it easy to identify the surgical instrument.

As with the second embodiment, the scissors blades 151, 151 of this embodiment are made by forging metallic materials, and therefore it is possible to prevent the abrasion or the spread of rubbed ares of the scissors blades 151, 151. In this way, it is possible to maintain the sharpness of the scissors forceps over a long period of time.

The scissors blades 151, 151 may be formed into any shapes. For example, although both of the pairs of the scissors blades 141, 141 and 151, 151 shown in Figs. 17, 18A, and 18B are movable, either one blade of the pairs of the blades 141 and 151 may be formed like the fixed blade 101 that is integrated with the leading end cover 21 of the scissors forceps of the second embodiment shown in Fig. 16.

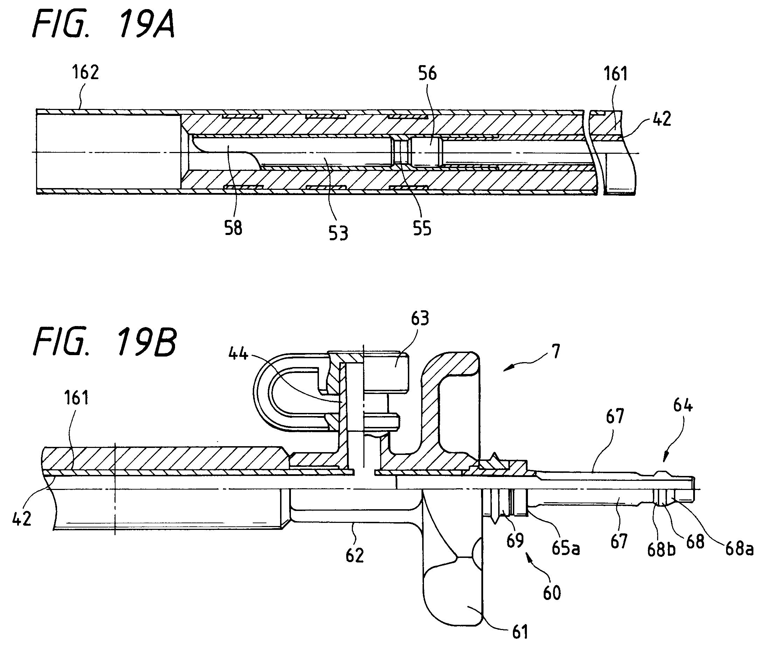

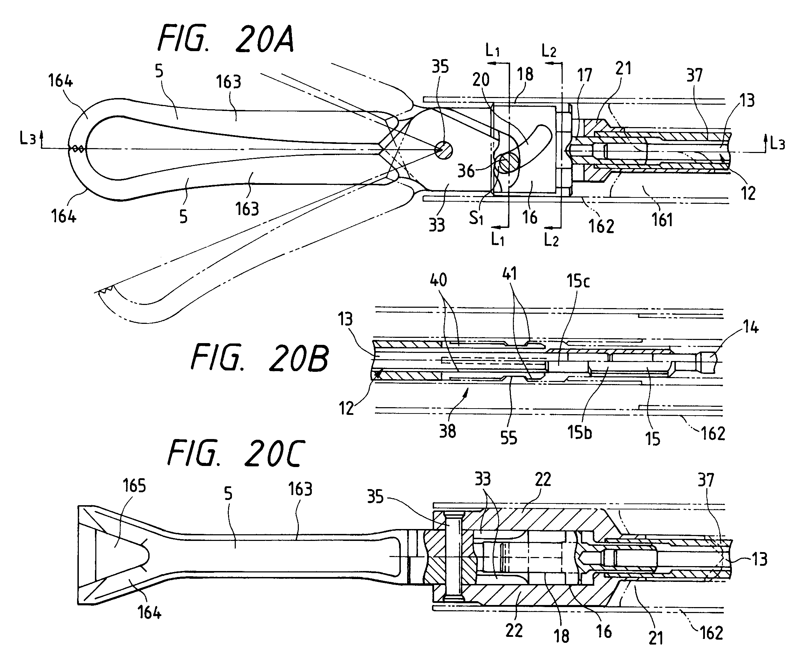

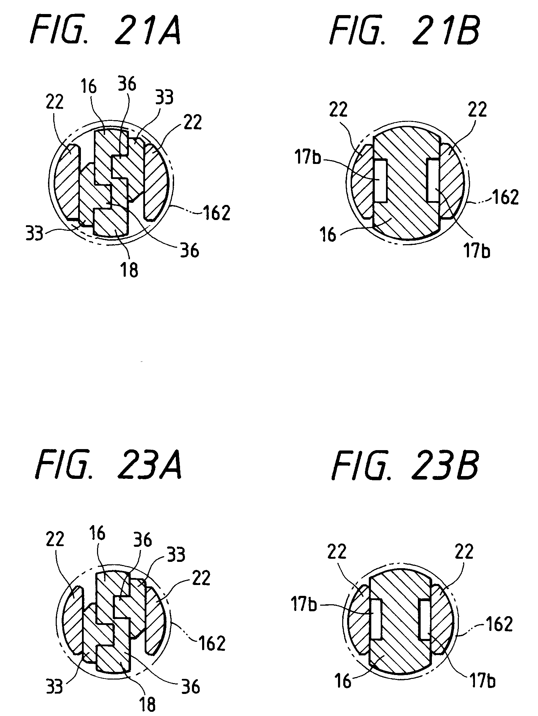

Figs. 19A to 21B show forceps according to a fifth embodiment of the present invention. Figs. 19A and 19B show the sheath unit 7 of this embodiment, and Figs. 20A to 20C and Figs. 21A and 21B show the treatment section drive unit 9 of this embodiment.

The sheath unit 7 of the surgical instrument of this embodiment similar to the sheath unit of the first embodiment has its configuration changed as it is shown in Figs. 19A and 19B. Specifically, the outer peripheral surface of the inner pipe 42 is covered with a large-diameter pipe 161. The leading end of this large-diameter pipe 161 extends to the outside of the leading end of the connection pipe 53, as shown in Fig. 19A. In other words, the connection pipe 53 is also covered with this large-diameter pipe 161.

The outer peripheral surface of this large-diameter pipe 161 is further covered with an insulating tube 162. The outer diameter of this insulating tube 162 is set to the same as the outer diameter of the large-diameter pipe 161, and the outer peripheral surface of the leading end portion of the large-diameter pipe 161 is covered with this insulating tube 162. Here, the outer diameter of the large-diameter pipe 161 and the insulating tube 162 is set so as to be insertable into the inside of a tracheal mantle tube (not shown) or a surgical instrument insertion channel of an endoscope (not shown).

The outer diameter of the leading end cover 21 of the treatment section drive unit 9 is set relatively large to such an extent that the leading end cover 21 can be housed in the large-diameter insulating tube 162 of the sheath unit 7, as shown in Figs. 20A to 20C. The outer diameter of the forceps opening and closing member 16 is also set large in agreement with the shape of the leading end cover 21.

The outer diameter of a main body 163 of each of the grasping member 5 is set relatively large to such an extent that the main body 163 can be housed in the large-diameter insulating tube 162 of the sheath unit 7. Curved sections 164 which are curved inwardly are provided at the leading ends of the grasp member main body 163, as shown in Fig. 20A. A substantially triangular window 165 is further formed in the leading end portion of each curved section 164, as shown in Fig. 20C. This window is intended to improve the grasping properties of the forceps when the forceps grasp the tissue H by letting deformed tissue go through the substantially triangular windows 165.

As with the first embodiment, each of the grasping members 5,5 is made by forging metallic materials, and hence the fiber flow appears in the longitudinal direction of the grasping members 5, 5. When the forceps grasp the tissue H, an intermediate portion of each of the grasping members 5, 5 resiliently deforms, whereby the bending action of the grasping members prevents the tissue H from undergoing an excessive grasping force.

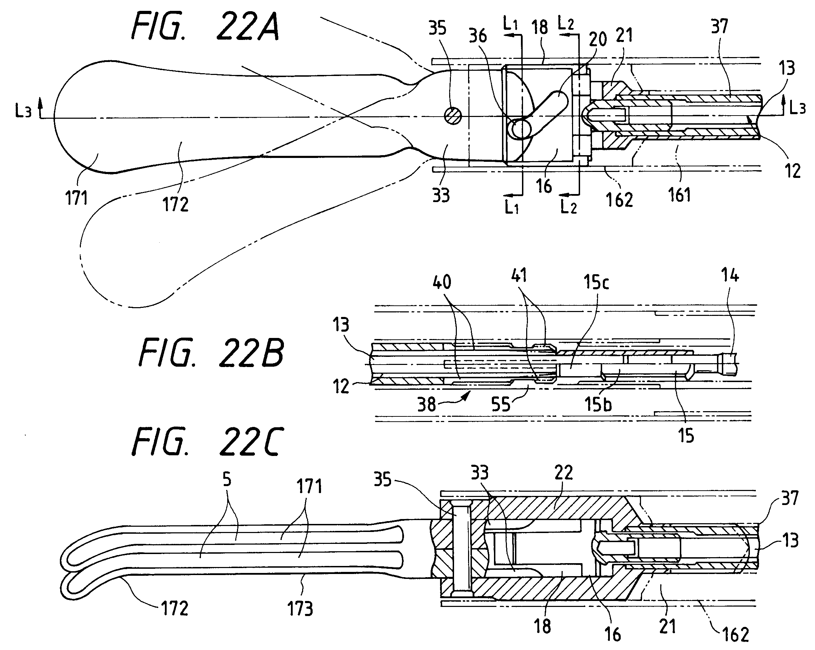

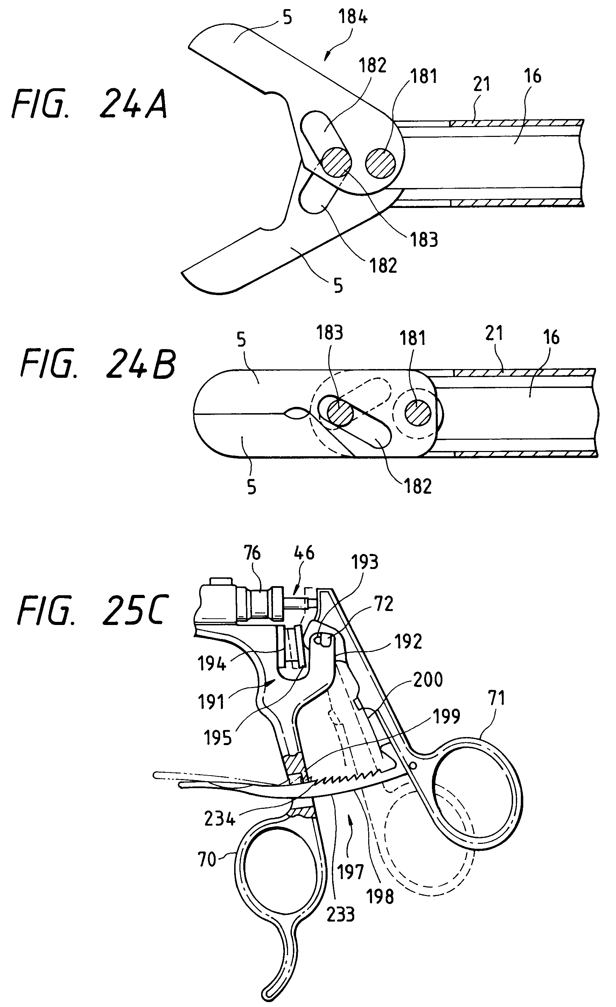

Figs. 22A to 22C and Figs. 23A and 23B show forceps according to a sixth embodiment of the present invention. The forceps of this embodiment are substantially the same in configuration as the forceps of the fifth embodiment shown in Figs. 20A to 20C. However, instead of the grasp member main body 163 of the fifth embodiment, a pair of pushing-aside elements 171 that are laid one over the other like the scissors of the fourth embodiment shown in Fig. 18 are provided. These pushing-aside elements 171, 171 are used for pushing-aside the tissue H when the pushing-aside elements 171, 171 are opened. The pushing-aside elements are made by forging metallic materials.

A curved section 172 is provided at the leading end portion of each of the pushing-aside elements 171. A pushing-aside surface 173 of each of the pushing-aside element 171 is crimped so as to ensure the pushing-aside the tissue H by preventing a slip of the tissue H. Each of the pushing-aside elements 171 is forged in such a way that the fiber flow appears in the longitudinal direction of the pushing-aside element 171 in the same way as previously mentioned, and hence the pushing-aside element 171 has appropriate elasticity. This pushing-aside element 171 deforms in agreement with the shape of the tissue H which needs be pushed-aside, or it deflects upon receipt of an excessive force, as a result of which an excessive force is not transmitted to the tissue H.

Figs. 24A and 24B show forceps according to a seventh embodiment of the present invention. This embodiment is different from the first embodiment in that the configuration of the treatment section 6 is changed. In other words, in this embodiment, the rear end portions of the pair of grasping members 5, 5 are connected to the forceps opening and closing member 16 via a joint pin 181 in such a way as to be pivotally movable around the joint pin 181.

An elongated hole 182 for guiding the opening and closing action of the grasping member is formed in each of the grasping members 5. A caulking pin 183 is inserted in the elongated holes 182, 182 of the grasping members 5, 5, and the caulking pin 183 is caulked between the pair of support arms 22, 22 of the leading end cover 21.

The elongated holes 182 move along the caulking pin 183 as the forceps opening and closing member 16 advances or recedes. As a result of this, the grasping members 5, 5 pivotally move around the joint pin 181, thereby constituting a link mechanism 184 which opens and closes. The other parts of forceps of this embodiment are substantially the same in configuration and operation as the corresponding parts of the forceps of the first embodiment.

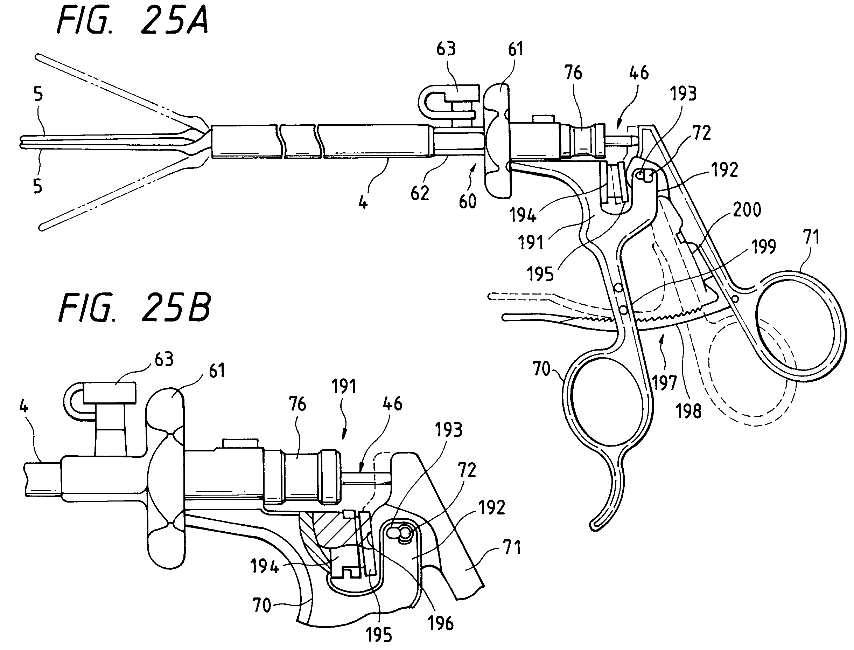

Figs. 25A and 25B show forceps according to an eighth embodiment of the present invention. The forceps of this embodiment is provided with grasping force control means 191 for controlling an operation force, i.e., a grasping force for moving the movable operation handle 71 in a closing direction with respect to the fixed handle 70 of the first embodiment.

Specifically, a substantially J-shaped projection 192 is provided at the joint between the movable operation handle 71 and the fixed handle 70. An elongated hole 193 into which the joint screw 72 is inserted is formed in the leading end portion of the projection 192, and this joint screw 72 acts as the pivot of the rotation of the movable operation handle 71. This joint screw 72 is movable to and fro along the elongated hole 193 together with the movable operation handle 71 with respect to the fixed handle 70.

This fixed handle 70 is further provided with a rubber member 194 for controlling the grasping force. A rubber cover 195 attached to the rubber member 194 is brought into contact with a slide surface 196 of the movable operation handle 71.

If the force for actuating the grasping members 5, 5 of the treatment section 6 in a closing direction is not exerted on the movable operation handle 71, the movable operation handle 71 is pushed toward the rear end portion of the elongated hole 193 by the resilient force of the rubber member 194. If the force for pushing the grasping members 5, 5 of the treatment section 6 in the closing direction is exerted on the movable operation handle 71, the rubber member 194 is pushed forward by the slide surface 196 of the movable operation handle 71, whereby the movable operation handle 71 slides along the elongated hole 193 to the front leading end portion thereof.

At this time, the elastic modulus of the rubber member 194 is arbitrarily set with respect to the elastic modulus of the grasping members 5, 5. Specifically, it is possible to set the elastic modulus of the rubber member 194 in such a way that the rubber member 194 deforms after the grasping members 5, 5 have been completely closed or that the rubber member 194 deforms before the grasping members 5, 5 deform. As a result of this, in addition to the control of the grasping force by means of the elasticity of the grasping members 5, 5, the control of the grasping force by means of the elastic force of the rubber member 194 becomes possible. The control of the grasping force is effective in preventing the tissue H from being damaged.

The elastic force of the rubber member 194 is not substantially used when the grasping members 5, 5 are opened. For example, on the assumption that the forceps have no rubber member 194, that the elastic modulus of the grasping members 5, 5 must be set very small to grasp the tissue H intact, and that the mechanical strength of the grasping members 5, 5 is too small to exfoliate the tissue H without deforming, it is possible to set the elastic modulus of the grasping members 5, 5 in the range where the exfoliating action of the grasping members 5, 5 is performed smoothly by the use of the elastic forces of the grasping members 5, 5 and the rubber member 194 in order to grasp tissue H intact.

The operation section unit 3 of the present embodiment is provided with a rachet mechanism 197. In this rachet mechanism 197, the amount of engagement between a rachet 198 attached to the movable operation handle 71 and engaging pawls 199 provided on the fixed handle 70 increases as the movable operation handle 71 moves in a closing direction. Reference numeral 200 designates a leaf spring member which engages with the base end portion of the rachet 198.

Conventionally, the amount of engagement between the rachet 198 and the engaging pawls 199 cannot be increased any further without deforming the tissue H if the grasping members 5, 5 are closed or if the tissue H is grasped between the grasping members 5, 5. To completely grasp the tissue H, it is necessary to further move the rachet 198 in the direction, in which the amount of engagement increases, from the position of the engagement between the rachet 198 and the engaging pawls 199 where the tissue H was grasped. As a result of this, an unnecessarily strong closing force acts on the movable operation handle 71, whereby an unnecessarily strong force is further exerted on the grasping members 5, 5, which might result in damaged tissue H. Contrary to this, in the case of this embodiment, the grasping members 5, 5 and the rubber member 194 deform so as to absorb the excessive grasping force, and hence it is possible to increase the amount of engagement between the rachet 198 and the engaging pawls 199 with a constant grasping force even while the tissue H is grasped or the grasping members 5, 5 are closed, thereby realizing stable and reliable grasping of the tissue.

Fig. 25C shows a modified example of the forceps shown in Figs. 25A and 25B. Pawls 233 of the rachet 198 are formed in such a way that the rachet 198 engages with the engaging pawls 199 after the rubber member 194 has deformed. In other words, a start position 234 of the pawls 233 is shifted toward the movable operation handle 71 compared with the start position which is ordinarily set.

It is set that the rubber member 194 deforms if the movable operation handle 71 is closed further after parts of the grasping members 5, 5 (for example, the leading ends of the grasping members 5, 5) have come into contact with each other during the closing action. As a result of this, the rachet 198 starts to engage with the engaging pawls 199 after the tissue H has been grasped by the grasping members 5, 5 and the rubber member 194 has deformed.

For common forceps having a rachet for operation use, it is necessary to release the rachet every time grasping members are opened. For this reason, such a rachet obstructs operation which needs repeated opening and closing action of the grasping members (for example, operation for gently grasping tissue or applying a force to the grasping members chiefly in an opening direction).

However, according to this embodiment, the rachet 198 starts to operate after the rubber member 194 has deformed and the grasping members 5, 5 have closed (that is, when the user wants to firmly hold the tissue). The rachet 198 does not operate by such gentle grasping action that when the leading ends of the grasping members come into contact with the tissue. Even if the opening and closing action of the grasping members are repeated, this rachet provides easy operation.

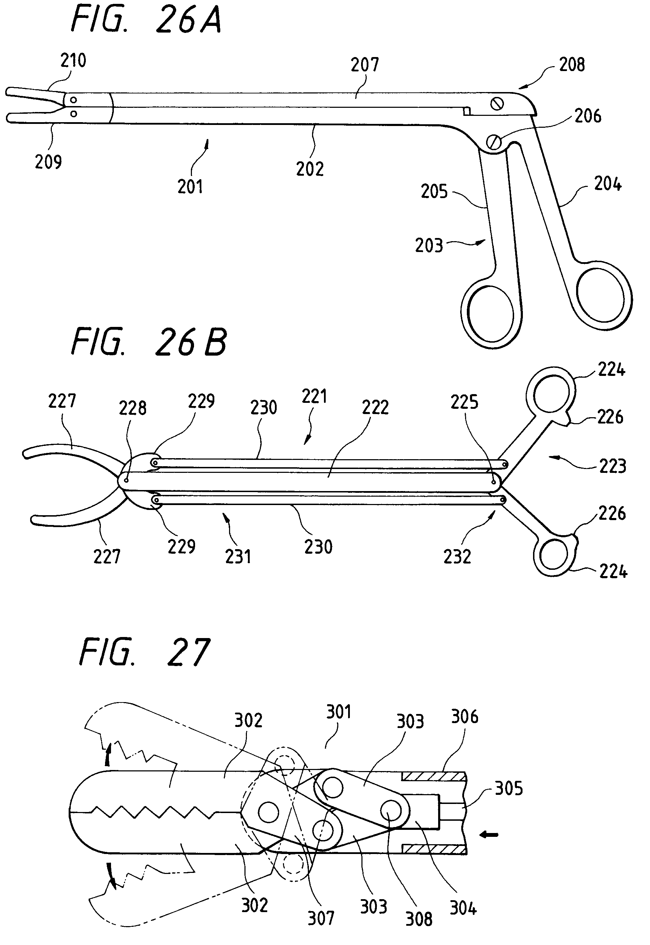

Fig. 26A shows forceps according to a ninth embodiment of the present invention. In this embodiment, an insertion section 202 of a surgical instrument 201 is provided with a fixed handle 204 of an operation section 203 in an integrated fashion. A movable operation handle 205 is rotatably connected to the fixed handle 204.

An upper portion of the movable operation handle 205 is connected to the base portion of an external rod member 207 which serves as transmission means, whereby link type conversion means 208 are formed.

A fixed lower blade 209 and a movable upper blade 210 are attached to the leading end portion of the insertion section 202. The lower fixed blade 209 is formed together with the leading end portion of the insertion section 202 in an integrated fashion. The lower fixed blade 209 is further connected to the leading end portion of the external rod member 207 via a link type treatment member drive mechanism. As with the first embodiment, the movable blade 210 is opened and closed as a result of the opening and closing action of the movable operation handle 205.

The upper movable blade 210 is made by forging metallic materials. For this reason, when the tissue H is grasped between the lower fixed blade 209 and the upper movable blade 210, the upper movable blade 210 deflects, as a result of which the tissue is grasped intact in the same manner as in the second embodiment.

Fig. 26B shows forceps according to a tenth embodiment of the present invention. Two movable operation handles 224, 224 of an operation section 223 are pivotally connected to the end on the handle side of an insertion section 222 of a surgical instrument 221 via a common pivot pin 225. A rachet 226 is formed in each of the movable operation handles 224. The movable operation handles 224 are made by forging metallic materials in such a way that the fiber flow appears in the longitudinal direction of the movable operation handles 224, 224 which connects between the pivot pin 225 and the ratchets 226.

A pair of grasping members 227, 227 are pivotally connected to the leading end of the insertion section 222 via a common pivot pin 228. Further, the leading end of an external rod member 230 which serves as transmission means is connected to an arm section 229 on the base side of each grasping member 227. The base portion of each external rod member 230 is connected to each of the movable operation handles 224. The grasping members 227, 227 are formed by forging metallic materials in such a way that the fiber flow appears in the longitudinal direction of the grasping members 227.

As a result of this, a treatment member drive mechanism 231 is formed between the external rod members 230, 230 and the arm sections 229, 229 of the pair of grasping members 227, 227. Further, conversion means 232 is formed between the external rod members 230, 230 and the movable operation handles 224, 224. The pair of grasping members 227, 227 open and close in response to the opening and closing action of the movable operation handles 224, 224.

The surgical instrument 221 with the above mentioned construction has superior deep approaching properties as well as ease of use which is the same as that of surgical forceps. Particularly, this surgical instrument 221 is effective for use in pleural cavity operation without the necessity of a minor surgical operation, or suspended type abdominal cavity mirror operation using a mirror.

In this embodiment, the grasping members 227, 227 and the movable operation handles 224, 224 are formed by forging metallic materials. For this reason, as with the first embodiment, if an excessive grasping force is exerted no the grasping members 227, 227, the movable operation handles 224, 224 deflect in addition to the deflection of the grasping members 227, 227, as a result of which the excessive grasping force is absorbed by the defecting action of these members. Therefore, it is possible to realize the grasping of the tissue H without damage. In the case of this embodiment, the ratchets 226 are the same in shape as common surgical forceps, which provides ease of use and facilitated maintenance such as cleansing and sterilization.

Fig. 27 is grasp type forceps according an eleventh embodiment of the present invention, wherein the connection means of the treatment member drive mechanism is made of a link mechanism. Fig. 27 shows a treatment section 301 of the forceps. The treatment section 301 is made up of grasping members 302, link members 303, a forceps opening and closing member 304, an actuation shaft 305, and a leading end cover 306. The grasping members 301 are pivotally supported by the leading end cover 306. One end of each of the grasping members 301 forms a connection arm 307, and the connection arms 307 are pivotally connected to the leading end of the actuation shaft 305 by means of the forceps opening and closing members 304 and the link members 303 which are provided at the leading end of the actuation shaft 305. The link members 303 are usually connected to the actuation shaft 305 by a joint pin 308. The treatment section 301 is further connected to the sheath unit and the operation section drive unit as shown in Fig. 1, and the grasping members 301 can open and close by causing the actuation shaft 305 to move to and fro. At least one of all the parts constituting the treatment section is made by forging.

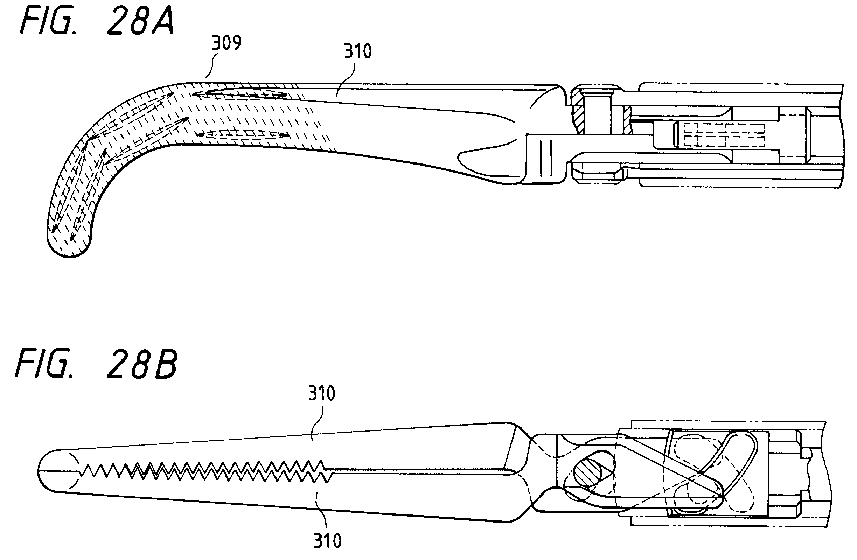

Figs. 28A and 28B show a surgical instrument for operation purposes according to a twelfth embodiment of the present invention, in which separation type forceps are used as the grasping member. This embodiment is different from the first embodiment in that separation members are used instead of the grasping members shown in Fig. 2. Separation forceps 309 are mainly used for separating tissue from the living body. Since the separation forceps 309 are used for detailed operation, narrow forceps are often demanded. The forceps may be curved as shown in Fig. 28 in certain circumstances.

Even in the case of this embodiment, at least one of all the parts which form the treatment section including separation members 310 is made by forging. The separation members 310 are narrow in shape, and hence the manufacture of the separation members by forging is particularly effective. Furthermore, it is difficult and laborious to manufacture the separation members 310 as shown in Fig. 28 by machining. Contrary to this, it is possible to easily manufacture the separation members by forging.

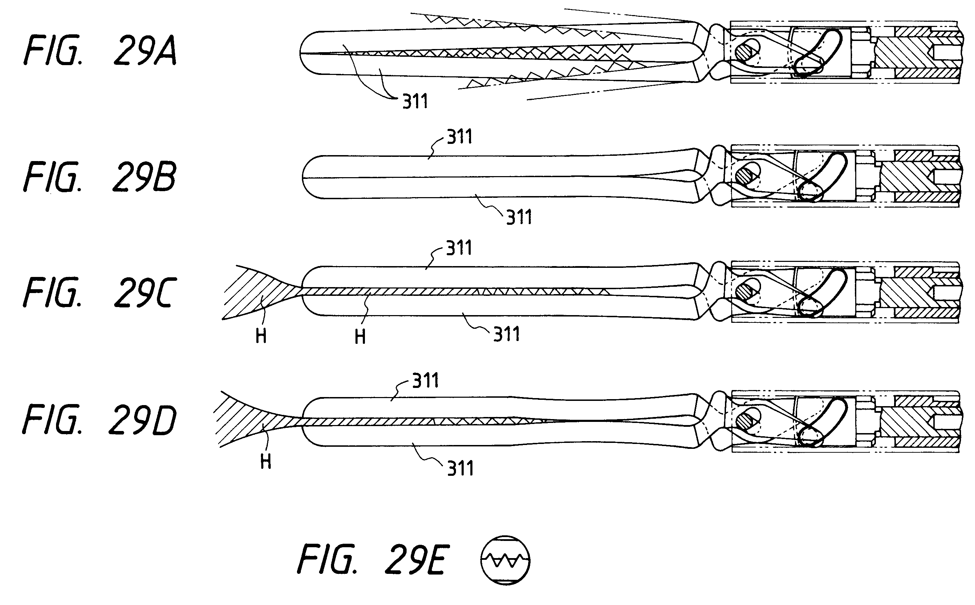

Figs. 29A to 29E show forceps according to a thirteenth embodiment of the present invention.

In the embodiment shown in Figs. 29A to 29E, grasping members 311 are manufactured by forging chiefly in order to provide the grasping members with elasticity.

When the grasping members 311 are progressively closed, the leading end portions of the grasping members 311 first come into contact with each other, as shown in Fig. 29A. If the grasping members 311 are further rotated in the closing direction, the entire grasping members 311 or a part thereof deforms, so that a contact area between the grasping members 311 increases as shown in Fig. 29B. When the grasping members 311 actually grasp tissue H, the grasping members 311 can grasp the tissue H while they are substantially parallel with each other, as shown in Fig. 29C. If the grasping members 311 are further rotated in the closing direction, a part of the grasping members 311 deforms as shown in Fig. 29D.

If the cross sectional area of a part of unillustrated parts of the grasping members is made smaller than the cross sectional areas of the other parts, the grasping members 311 will become deformable more easily.

As shown in Fig. 29E, longitudinal engaging crimps are formed in the contact surfaces of the grasping members 311 to prevent the slip of tissue.

To manufacture grasping members which have a plenty of elasticity like the grasping members of this embodiment, the grasping members were conventionally manufactured by any one of the following methods.
1: The grasping members are made of common stainless steel, and at least a part of the grasping member is machined in the shape of a thin plate or an approximate rod so that it can resiliently deform as much as possible.
2: The treatment member is manufactured by machining spring steel which has a plenty of elasticity.
3: The grasping member is manufactured by connecting a member having a plenty of elasticity (for example, a leaf spring material) to the treatment member without elasticity.

In the case of the first method, the amount of deformation of the grasping members is small as a result of the use of the common stainless steel, and also it was difficult to machine the stainless steel into the shape of a thin plate or a substantial rod. In the case of the second method, the spring steel material is not suitable for machining, and hence it is impossible to machine the spring steel material into a complicated structure. In the case of the third method, it takes much time to connect separately manufactured parts with together. The present invention is constructed so as to eliminate these drawbacks in the prior art, and the following advantageous results are obtained.

Specifically, (1) forging permits elastic materials represented by spring steel to be processed, and hence grasping members possessing a plenty of elasticity can be manufactured. (2) The present invention does not need the previously mentioned connection of individually manufactured parts, and hence it becomes easier to manufacture the grasping members. (3) Forging needs neither machining nor hardening. (4) The leading end portions of the grasping members of this embodiment inevitably come into contact with each other when the grasping members close. Therefore, even in the case of thin tissue, the grasping members can easily grasp the tissue. (5) The contact area between the grasping members becomes substantially parallel as the grasping members are closed, whereby the tissue becomes less likely to slip. (6) If a force more than required is exerted on the grasping members, the grasping members deform, whereby the excessive force does not act on the tissue. In this way, it is possible to prevent the tissue from being damaged, and hence the tissue can be grasped intact.

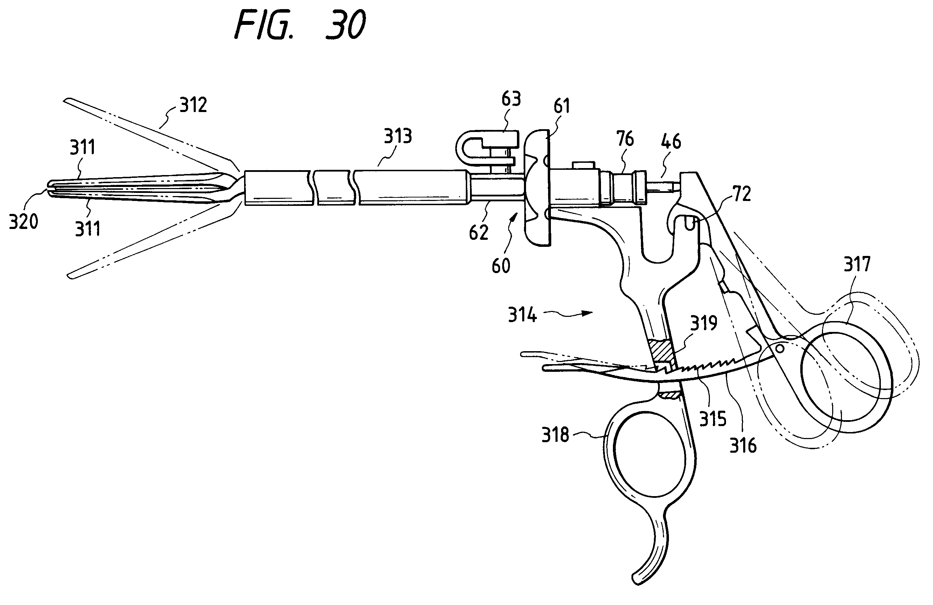

With reference to Fig. 30, the operation section of the forceps according to the thirteenth embodiment will now be described.

A treatment section 312 is connected to an operation section unit 314 via sheath unit 313. The operation section unit 314 is provided with a rachet 316 having teeth 315. When a movable handle 317 is rotated in the closing direction (the grasping member 311 also moves in the closing direction in response to the rotation of the movable handle 317), the amount of engagement between the rachet 316 and engaging pawls 319 of a fixed handle 318 increases.

After leading end portions 320 of the grasping members 311 have come into contact with each other (the grasping members have closed slightly further compared with the grasping members shown in Fig. 29A), the position of the teeth 315 is set in such a way that the teeth 315 of the rachet 316 engage with engaging pawls 319.

The rachet 316 is not actuated before the leading end portions 320 come into contact with each other and the grasping members 311 slightly deform. When the grasping members 311 deform to close as shown in Figs. 29B to 29C (when the user wants to firmly grasp the tissue), the rachet 316 is actuated.

By virtue of such a configuration, the following advantageous results are also obtained in addition to the previously mentioned effects resulting from the manufacture of the grasping members by forging. In other words, when the grasping members deform and close (in other words, the user wants to firmly grasp the tissue) in this embodiment, the rachet is actuated. The rachet is not actuated by such gentle grasping action that the leading ends of the grasping members come into contact with the tissue. This rachet provides easy operation particularly when the opening and closing action of the grasping members are repeated.

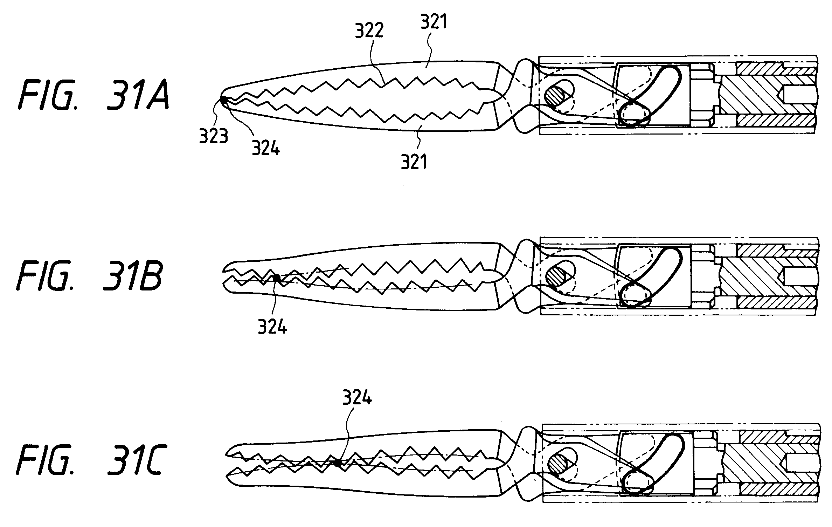

Figs. 31A to 31C show grasp type forceps according to a fourteenth embodiment of the present invention. In this embodiment, grasping members 321 are manufactured by forging mainly in order to provide the grasping members with elasticity. In this embodiment, teeth 322 of the grasping member 321 are arranged in an approximately bow-shaped pattern, and the hardness of the grasping member 321 is set low. A contact point 324 shifts from (a), (b), and (c) in that order from the leading end of the grasping member as the grasping members 321 are further rotated in a closing direction after leading end portions 323 of the grasping members 321 have been brought into contact with each other. According to the fourteenth embodiment, in addition to the previously mentioned advantageous effects resulting from the manufacture of the grasping members by forging, it is easy to grasp a part of the tissue because the grasping members 321 are closed remaining in partial contact with each other when the grasping members are closed. Such forceps are suitable for detailed operation.

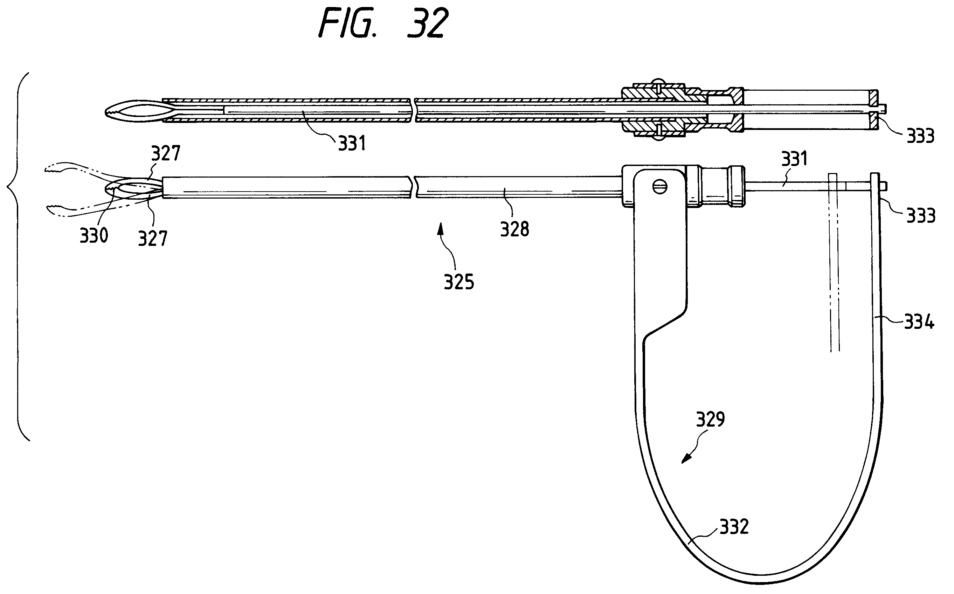

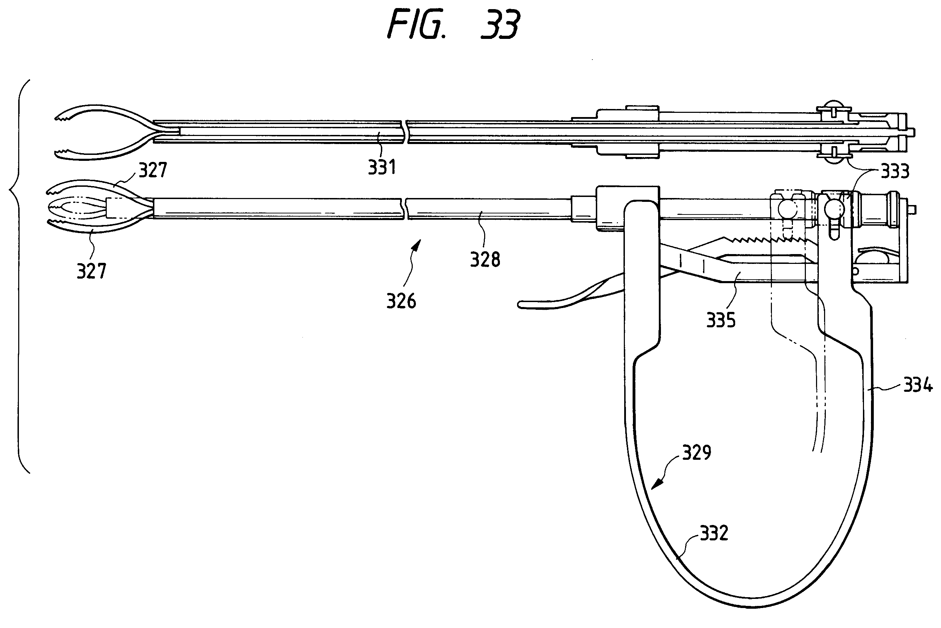

Figs. 32 to 37B show a modified example of the surgical instrument for operation use having another type of drive mechanism. Forceps for operation use 325 and 326 shown in Figs. 32 and 33 are made up of treatment members 327, an insertion section 328 made of a hollow pipe, and an operation section 329. The treatment members 327 have treatment sections 330 at the leading end portions thereof.

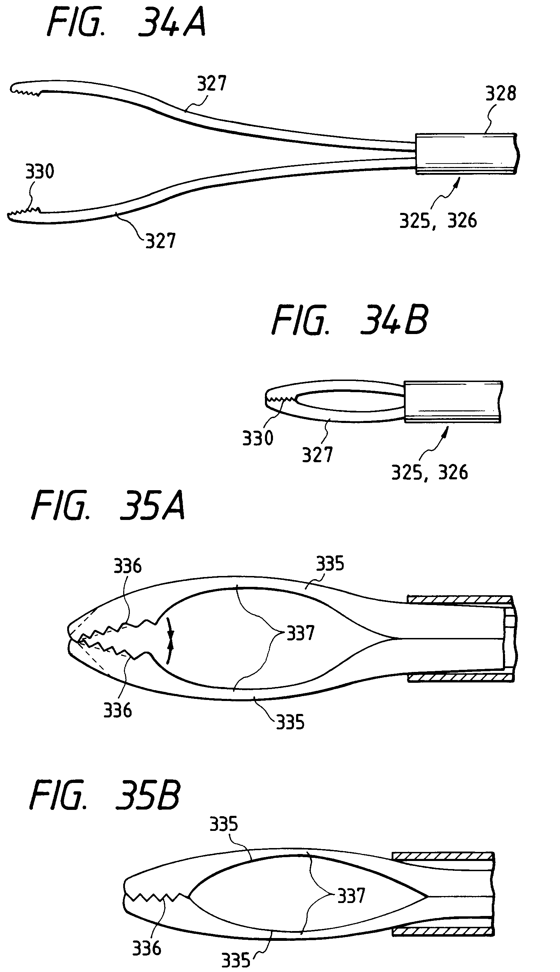

In Fig. 32, one end of the insertion section 328 is fixed to the operation section 329, and the opposite ends of the treatment members 327 with respect to the treatment sections 330 are connected to an actuation shaft 331 which is slidable within the insertion section 328. The opposite end of the actuation shaft 331 with respect to the end connected to the treatment members 327 is connected to a joint section 333 of a handle 332 which is made of a leaf spring. The treatment members 327 usually have a shape designated by a two-dot chain line (or a shape shown in Fig. 34A). When the handle-side ends of the treatment members 327 are covered with the leading end side of the insertion section 328, the treatment members 327 are closed (Fig. 34B). Contrary to this, when the treatment members 327 project out of the insertion section 328, the treatment members 327 resumes the shape designated by two-dot chain line (Fig. 34A).

The treatment members 327 are manufactured by forging mainly in order to provide the treatment members with elasticity.

Since the treatment members open and close by utilization of their elasticity, it is particularly desirable to manufacture the treatment members of material possessing a plenty of elasticity. For this reason, the treatment members were conventionally manufactured by the following method:
1. The treatment member is manufactured by machining spring steel which has a plenty of elasticity.
2. The treatment member is manufactured by connecting a member having a plenty of elasticity (for example, a leaf spring material) to the treatment member without elasticity.

In the case of the first method, the spring steel material is not suitable for machining, and hence it is impossible to machine the spring steel material into a complicated structure. In the case of the second method, it takes much time to connect separately manufactured parts with together.

According to the present embodiment, when the handle 332 is closed by pushing the rear end portion 334 of the handle 332 in the direction of the leading end of the forceps, the treatment members 327 connected to the actuation shaft 331 are released from the insertion section 328, whereby the treatment members 327 open.

The following advantageous results are obtained as a result of the manufacture of the treatment members by forging.
(1) Forging permits elastic materials which include spring steel to be processed, and hence treatment members possessing a plenty of elasticity can be manufactured.
(2) The present invention does not need the previously mentioned connection of individually manufactured parts, and hence it becomes easier to manufacture the grasping members.
(3) Forging needs neither machining nor hardening.
(4) If a force more than required is exerted on the treatment members, the treatment members deform, whereby the excessive force does not act on the tissue. In this way, it is possible to prevent the tissue from being damaged, and hence the tissue can be grasped intact.

In the embodiment shown in Fig. 33, the opposite end of the insertion section 328 to the end connected to the treatment members 327 is connected to the joint section 333 of the handle 332 made of a leaf spring. The opposite ends of the treatment members 327 to the ends having the treatment section 330 are connected to the actuation shaft 331 which is slidable within the insertion section 328. Further, the opposite end of the actuation shaft 331 to the end connected to the treatment members 327 is connected to a main body 335 of the operation section 329. The main body 335 has a rachet which carries out positioning by engaging with the joint 333 of the handle 332. The shape of the treatment members 327 is the same as the previous example shown in Fig. 32.

In this embodiment, the treatment members 327 usually project out of the insertion section 328. When the handle 332 is closed by pushing the rear end section 334 of the handle 332 in the direction of the leading end of the forceps, the treatment members 327 are covered with the insertion section 328, whereby the treatment members 327 are closed. This example is the same as the previous example shown in Fig. 32 in that the treatment members 327 and the insertion section 328 move relatively to each other, and therefore the same advantageous effects are obtained.

Whether to make the treatment members 327 usually opened or closed depends on the selection of the shape of the handle 332. Further, the operation section 329 shown in these modified examples is an illustrative example. The operation section 329 may be replaced with, for example, the combination of a fixed handle and a rotatable handle.

Figs. 34A to 37B show illustrative examples of a leading end section. The illustrative examples which will be described below may be combined with either of the examples shown in Figs. 32 and 33.

Grasping members, scissors members, and pushing-aside members of the following example a manufactured by forging in order to provide them with elasticity.

Figs. 34A and 34B show an example of grasp type forceps. Fig. 34A shows the forceps when opened, and Fig. 34B shows the forceps when closed.

Figs. 35A and 35B show an example of grasp type forceps. Fig. 35A shows the forceps when the leading ends of grasping members of the forceps are in contact with each other, and Fig. 35B shows the forceps when the grasping members are closed. Each of grasping members 335 has a grasp section 336, and an intermediate portion of the grasping member 335 between both ends thereof has a reduced cross sectional area compared with the other portions thereof.

The leading ends of the grasp sections 336 come into contact with each other as the grasping members 335 are closed. Thereafter, the grasping members 335 are progressively closed as if the grasp sections 336 pivotally move around the leading ends as the grasping members 335 are further closed. With this configuration, the grasping members 335 become easier to deform by virtue of the reduced intermediate portions, and the tissue can be reliably grasped by the pivotal closing action of the grasp sections.

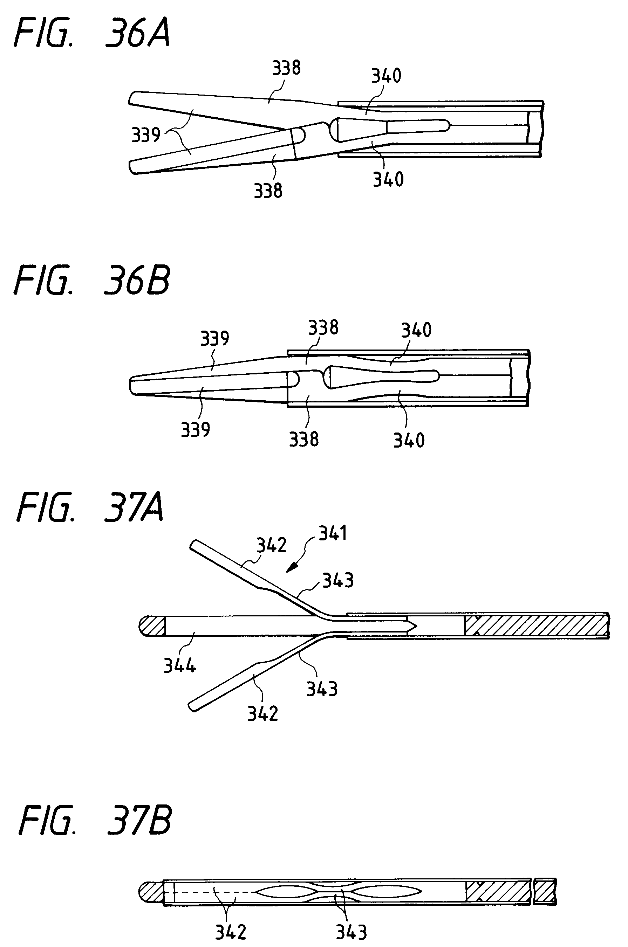

Figs. 36A and 36B show scissors type forceps. Fig. 36A shows the forceps when opened, and Fig. 36B shows the forceps when closed. Each of scissors members 338 has a blade 339, and an intermediate portion 340 of each scissors member 338 is formed into the shape of a rod so as to be easily deformable by reducing the cross sectional area of the intermediate portion 340.

By virtue of the rod shaped intermediate portion 340, the scissors members 338 are deformable not only in a vertical direction but also in a thicknesswise direction of the blades 339. Therefore, it is possible to keep the blades rubbing against each other, and also possible to maintain the sharpness of the forceps over a long period of time.

Figs. 37A and 37B shows pushing-aside elements. Each of pushing-aside members 341 has a pushing-aside section 342, and an intermediate portion 343 of the pushing-aside member 341 has a reduced cross sectional area. One end of the pushing-aside member 341 is connected to an actuation shaft 344. The advantageous effects resulting from the configuration of this embodiment are the same as those of the grasp type forceps.

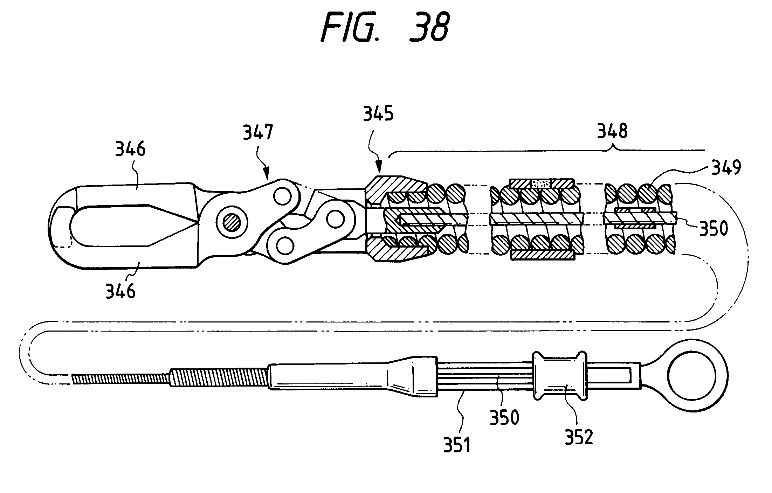

Fig. 38 shows the insertion section formed from a coil so as to have soft properties. Grasp forceps 345 are provided with a treatment section 347 which has grasping members 346, and a link mechanism which actuate the forceps is the same as the link mechanism shown in Fig. 27. A sheath 348 is made of a coil 349, and an operation wire 350 is provided within the coil 349. One end of the sheath 348 is connected to an operation section 351, and an operation wire 350 is connected to a slider 352. At least one of the parts which form the treatment section 347 is made by forging.

The grasping members 346 are opened or closed as a result of the movement of the operation wire 350 to and fro associated with the sliding action of the slider 352. As one example of the use of the forceps of this embodiment, the forceps are usable for treatment if they are inserted into an endoscope.

The previously mentioned combination of the drive mechanism, the leading-end opening and closing member (for example, the grasping members and the separation members), the insertion section, and the operation section is an illustrative example, and the present invention is not limited to this.

An illustrative example of the rachet mechanism will now be described.

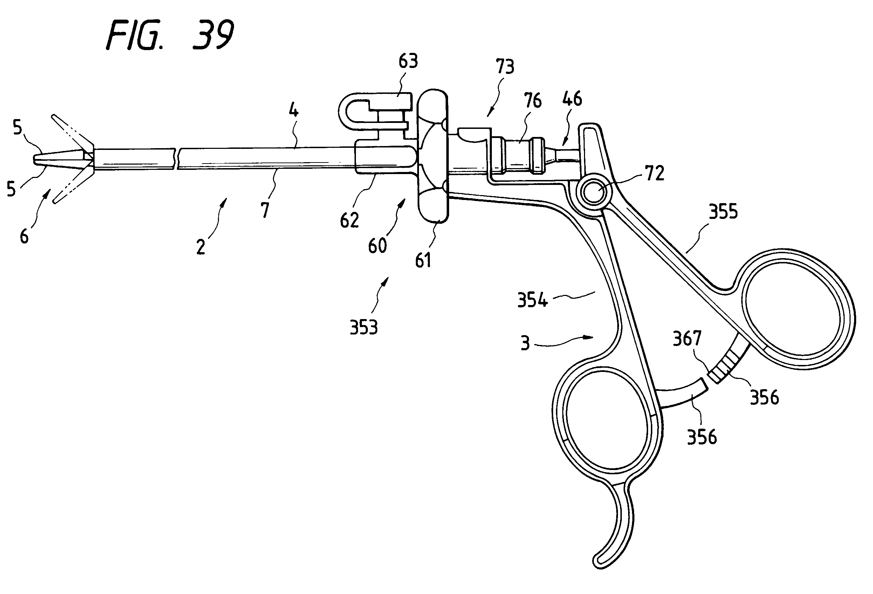

Fig. 39 shows grasp type forceps. Forceps 353 are provided with a fixed handle 354 and a movable handle 355, and these handles are manufactured by forging or another method so as to become resiliently deformable. These handles 355 and 354 are provided with ratchets 356, respectively. These ratchets 356 engage with each other by teeth 367 formed in each rachet. The length of the ratchets 356 is determined in such a way that the ratchets 356 do not engage with each other when grasping members 5 are closed, and that the ratchets 356 engage with each other as a result of the resilient deformation of the movable handle 355 and the fixed handle 354 caused by further closing the movable handle 355 from the state as shown in Fig. 39.

In this illustrative example, when the grasping members 5, 5 are closed as a result of the deformation of the handles 355 and 354 (that is, when the user wants to firmly grasp the tissue), the rachet is actuated. The rachet is not actuated by such gentle grasping action that the leading ends of the grasping members come into contact with the tissue. Even when the opening and closing action of the forceps are repeated, this rachet provides easy operation.

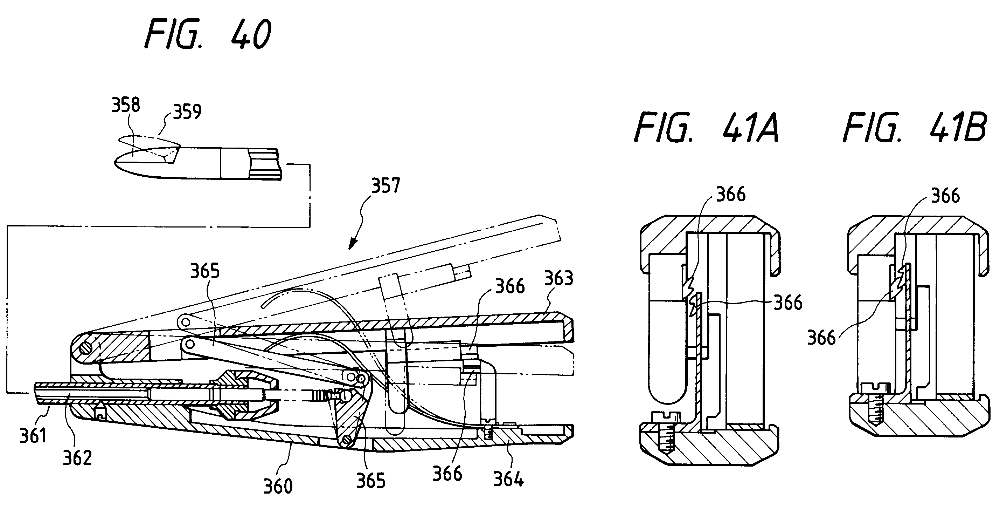

Fig. 40 shows a needle retainer. A needle retainer 357 is made up of a treatment section 359 having a grasping member 358, an operation section 360, and an insertion section 361 which connects the operation section 360 with the treatment section 359. The grasping member 358 opens and closes in response to the movement of an actuation shaft 362 which moves to and fro within the insertion section 361. The operation section 360 is provided with a movable handle 363, a fixed handle 364, and a link member 365 which turns as a result of the action of the handles. The end of the actuation shaft 362 is connected to the link member 365. The fixed handle 364 and the movable handle 363 are provided with ratchets 366, respectively.

The actuation shaft 362 is made of a spring member. When the grasping member 358 is in a normal closed state, the ratchets 366 do not engage with each other, as shown in Fig. 41A. However, the actuation shaft 362 resiliently extends as the movable handle 363 is closed, whereby the ratchets 366 engage with each other.

In the case of the needle retainer 357, a needle is frequently grasped again in order to correct the orientation of the needle, as required. The orientation of the needle is usually corrected by gently holding the needle, and at this time the rachet becomes unnecessary as it was in the case of the grasp type forceps. Conversely, if the needle is firmly grasped, the rachet becomes necessary. According to the present invention, as a result of the previously mentioned change of the position of the rachet, if the needle must be grasped firmly, the actuation shaft is deformed, whereby the ratchets engage with each other so as to close the grasping members. On the other hand, if the needle must be grasped gently, the ratchets do not engage with each other, and hence the ratchets do not obstruct the operation of the forceps.

The grasping members and the handles of the operation section as set forth above may be formed from resins such as polyether-ether-ketone (PEEK), polyetherimide, polysulfone, or polycarbonate.

Several embodiments of the invention have now been described in detail. It is to be noted, however, that these descriptions of specific embodiments are merely illustrative of the principles underlying the inventive concept. It is contemplated that various modifications of the disclosed embodiments, as well as other embodiments of the invention, will, without departing from the spirit and scope of the invention, be apparent to those who are versed in the art.

## Claims

1. A surgical instrument for operation purposes comprising:
an operation section operated by an operator;
a treatment section inserting into a living body to treat tissue;
an insertion section having a first end disposed at a side of said operation section and a second end disposed at a side of said treatment section; and
a treatment section drive mechanism operatively engaging between operation section and said treatment section, said drive mechanism driving said treatment section in response to operation of said operation section,
wherein at least a part of at least one of components constituting said treatment section and said treatment section drive mechanism is manufactured by forging metallic material.

2. The surgical instrument as defined in claim 1, wherein said treatment section drive mechanism comprises:
an elongating transmission section arranged substantially in parallel to said insertion section for transmitting the operation of said operation section to said treatment section by sliding relative to said insertion section; and
a connection section disposed between said transmission section and said treatment section.

3. The surgical instrument as defined in claim 2, wherein said connection section is manufactured by forging metallic material.

4. The surgical instrument as defined in claim 2, wherein said connection section has a cam mechanism comprising at least one cam groove and a slide pin which slides while engaging with said cam groove.

5. The surgical instrument as defined in claim 4, wherein said cam groove is disposed in any one of the first end of said transmission section and a connection member attached to said first end of the transmission section, and said slide pin is disposed in said treatment section.

6. The surgical instrument as defined in claim 4, wherein said cam groove is disposed in said treatment section, and said slide pin is disposed in any one of said first end of said transmission section and a connection member attached to said first end of said transmission section.

7. The surgical instrument as defined in claim 4, wherein said cam groove is disposed in said treatment section, and said slide pin is fixed to the insertion section, and any one of said first end of said connection section and a connection member attached to said first end of said connection section pivotally connects to said treatment section.

8. The surgical instrument as defined in claim 4, wherein said cam groove is disposed in said insertion section, and said slide pin is provided in said treatment section, and any one of said first end of said connection section and a connection member attached to said first end of said connection section is pivotally connected to said treatment section.

9. The surgical instrument as defined in claim 2, wherein said connection section has a link mechanism connecting to said treatment section and any one of said first end of said transmission section and a connection member disposed at said first end.

10. The surgical instrument as defined in claim 9, wherein said link mechanism has at least one link member and a connection pin connecting said link member.

11. The surgical instrument as defined in claim 1, wherein said treatment section has a first treatment member and a second treatment member which substantially moves pivotally relative to said first treatment section.

12. The surgical instrument as defined in claim 1, wherein said treatment section is manufactured by forging.

13. The surgical instrument as defined in claim 1, wherein at least a part of said treatment section is made of elastic material.

14. The surgical instrument as defined in claim 1, wherein said treatment section is made of stainless.

15. The surgical instrument as defined in claim 1, wherein said treatment section is made of titanium.

16. The surgical instrument as defined in claim 1, wherein said treatment section is made of aluminum.

17. The surgical instrument as defined in claim 11, wherein said treatment section comprises forceps teeth substantially in the form of sawtooth.

18. The surgical instrument as defined in claim 11, wherein said treatment section comprises scissors blades.

19. The surgical instrument as defined in claim 11, wherein said treatment section comprises forceps substantially bow-shaped teeth.

20. The surgical instrument as defined in claim 11, wherein said treatment section comprises a curved section which is curved in an inward direction, and a window is formed at the leading end of said curved section.

21. The surgical instrument as defined in claim 11, wherein said treatment section has a pair of pushing-aside elements.

22. The surgical instrument as defined in claim 21, wherein a curved section is formed at the leading end of each of said pushing-aside elements.

23. The surgical instrument as defined in claim 11, wherein said treatment section comprises a separation member.

24. The surgical instrument as defined in claim 1, wherein said operation section is manufactured by forging metallic material.

25. The surgical instrument as defined in claim 24, wherein said operation section is manufactured of metallic material by injection molding.

26. The surgical instrument as defined in claim 1, wherein at least a part of said operation section is covered with an insulating coating film.

27. The surgical instrument as defined in claim 26, wherein said insulating coating film is made of fluororesin.

28. The surgical instrument as defined in claim 1, wherein said operation section is made of insulating material.

29. The surgical instrument as defined in claim 1, wherein said operation section is made of synthetic resin.

30. The surgical instrument as defined in claim 1, wherein said operation section has a handle made of a leaf spring.

31. The surgical instrument as defined in claim 2, wherein said operation section comprises a first operation member connecting to said insertion section and a second operation section connecting to said transmission section of said treatment section drive mechanism, and said first and second operation members engage with each other by means of a rachet mechanism.

32. The surgical instrument as defined in claim 1, wherein said insertion section comprises a tubular sheath section for housing said treatment section drive mechanism.

33. The surgical instrument as defined in claim 32, wherein at least a part of said sheath section is made of an elastic member.

34. The surgical instrument as defined in claim 33, wherein a part of said sheath section is made of a coil.

35. The surgical instrument as defined in claim 2, wherein said treatment section drive mechanism comprises a treatment member opening and closing member disposed between said transmission section and said treatment section.

36. The surgical instrument as defined in claim 35, wherein said treatment member opening and closing member is covered with a cover which is made by forging metallic material.

37. The surgical instrument as defined in claim 2, wherein a pipe cover and a connection member are connected to one end of said transmission section.

38. The surgical instrument as defined in claim 37, wherein a connection ball which is made by forging metallic material and is removably connected to said operation section.

39. The surgical instrument as defined in claim 37, wherein at least a part of the outer peripheral of said connection member is covered with insulating material.

40. The surgical instrument as defined in claim 39, wherein said insulating material is a heat-shrinkable tube.

41. The surgical instrument as defined in claim 37, wherein said connection member comprises a small diameter section, and a breaking force of the small diameter section is smaller than the breaking force of the other members.

42. Forceps for operation use comprising:
a first elongating member inserting into a living body;
a second elongating member which is substantially parallel to said first elongating member and slidable relatively therewith;
an operation section disposed on a first end side of said first elongating member, said operation section connecting to said second elongating member; and
a treatment section disposed on a second end side of said first elongating member and opens and closes via said second elongating member in response to the operation of said operation section,
wherein said treatment section is formed by forging.

43. The forceps as defined in claim 42, wherein said first elongating member is fixed to said operation section, and only said second elongating member slides.

44. The forceps as defined in claim 42, wherein said second elongating member is fixed to said operation section, and only said first elongating member slides.

45. The forceps as defined in claim 42, wherein at least a part of said treatment section is made of elastic material.

46. The forceps as defined in claim 42, wherein said treatment section comprises an intermediate section a cross sectional area of which is smaller than the cross sectional areas of any other parts of said treatment section.

47. The forceps as defined in claim 42, wherein said second elongating member is made of a hard member.

48. The forceps as defined in claim 42, wherein said treatment section is made of stainless.

49. The forceps as defined in claim 42, wherein said treatment section is made of titanium.

50. The forceps as defined in claim 42, wherein said treatment section is made of aluminum.

51. The forceps as defined in claim 42, wherein said treatment section comprises a substantially turnable treatment member.

52. The forceps as defined in claim 42, wherein said grasping section comprises forceps teeth substantially in the form of sawtooth.

53. The forceps as defined in claim 42, wherein said treatment section comprises scissors blades.

54. The forceps as defined in claim 42, wherein said treatment section comprises forceps bow-shaped teeth.

55. The forceps as defined in claim 42, wherein said treatment section comprises a curved section which is curved in an inward direction, and a window is formed at the leading end of said curved section.

56. The forceps as defined in claim 42, wherein said treatment section comprises a pair of pushing-aside elements.

57. The forceps as defined in claim 56, wherein a curved section is formed at the leading end of each of said pushing-aside elements.

58. The forceps as defined in claim 42, wherein said treatment section comprises a separation member.

59. The forceps as defined in claim 42, wherein said operation section is manufactured by forging metallic material.

60. The forceps as defined in claim 42, wherein said operation section is manufactured of metallic material by injection molding.

61. The forceps as defined in claim 42, further comprising an insulating coating film for covering said operation section.

62. The forceps as defined in claim 61, wherein said insulating coating film is made of fluororesin.

63. The forceps as defined in claim 42, wherein said operation section is made of insulating material.

64. The forceps as defined in claim 42, wherein said operation section is made of synthetic resin.

65. The forceps as defined in claim 42, wherein said operation section comprises a handle made of a leaf spring.

66. The forceps as defined in claim 42, wherein said operation section comprises a first operation member connecting to said insertion section and a second operation section connecting to said transmission section of said treatment section drive mechanism, and said first and second operation members engage with each other by means of a rachet mechanism.

67. The forceps as defined in claim 42, wherein at least a part of said second elongating member is made of an elastic member.

68. The forceps as defined in claim 67, wherein a part of said second elongating member is made of a coil.

69. A surgical instrument for operation use including two elongating members which are substantially parallel to each other and insert into a living body, an operation section disposed on one side of said two elongating members, and a treatment section disposed on the other side of said two elongating members which operates via said two elongating members in response to operation of said operation section, the surgical instrument being characterized in that
at least a part of at least one of said two elongating members, said operation section, and said treatment section is made by forging.

70. A method of manufacturing a surgical instrument for use in operation including two elongating members which are substantially parallel to each other and insert into a living body, an operation section disposed on one side of said two elongating members, and a treatment section disposed on the other side of said two elongating members which operates as a result of relative sliding action of said two elongating members in response to the operation of said operation section, the surgical instrument manufacturing method being characterized by comprising the steps of:
machining at least a part of at least one of said two elongating members and said treatment section; and
forging at least a part of at least one of said two elongating members and said treatment section.
